# EUROPÄISCHE PATENTANMELDUNG

(11) **EP 2 605 017 A1**
(43) Veröffentlichungstag der Anmeldung: **19.06.2013**
(21) Anmeldenummer: 11194099.5
(22) Anmeldetag: 16.12.2011
(51) Int. Cl.: G01N 33/574

(54) **Markersequenzen für gynäkologisches Malignom und deren Verwendung**

(71) Anmelder: Protagen AG, 44227 Dortmund (DE)
(72) Erfinder: Lüking, Angelika, 44892 Bochum (DE); Höpfner, Annabel, 33615 Bielefeld (DE); Schulz-Knappe, Peter, 30966 Hemmingen (DE); Kowald, Axel, 44892 Bochum (DE); Scheer, Christian, 44149 Dortmund (DE); Fiegl, Heidelinde, 6082 Ellbögen (AT); Daxenbichler, Günter, 6020 Innsbruck (AT)
(74) Vertreter: Simandi, Claus

(57) **Zusammenfassung**

Die vorliegende Erfindung betrifft Markersequenzen für gynäkologisches Malignom und deren diagnostische Verwendung, eine diagnostische Vorrichtung enthaltend Markersequenzen für gynäkologischem Malignom insbesondere eine Anordnung und einen Proteinbiochip sowie deren Verwendung. Ferner betrifft die Erfindung Verfahren zur Identifizierung von Markersequenzen für gynäkologisches Malignom und zum Screenen von potentiellen Wirkstoffen zur Behandlung und Prävention von gynäkologischem Malignom mittels dieser Markersequenzen.

## Beschreibung

Die vorliegende Erfindung betrifft Markersequenzen für gynäkologisches Malignom und deren diagnostische Verwendung, eine diagnostische Vorrichtung enthaltend Markersequenzen für gynäkologisches Malignom insbesondere eine Anordnung und einen Proteinbiochip sowie deren Verwendung. Ferner betrifft die Erfindung Verfahren zur Identifizierung von Markersequenzen für gynäkologisches Malignom und zum Screenen von potentiellen Wirkstoffen zur Behandlung und Prävention von gynäkologischem Malignom mittels dieser Markersequenzen.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig.

Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Das Zervixkarzinom (Carcinoma cervicis uteri), auch Kollumkarzinom oder Gebärmutterhalskrebs genannt, ist ein bösartiger (maligner) Tumor des Gebärmutterhalses (Cervix uteri). Es ist weltweit der zweithäufigste bösartige Tumor bei Frauen. Histologisch handelt es sich in der Mehrheit der Fälle um ein Plattenepithelkarzinom. Die häufigste Ursache für ein Zervixkarzinom ist eine Infektion mit bestimmten Typen des humanen Papillomvirus (HPV). Das Zervixkarzinom verursacht zunächst keine Schmerzen, nur gelegentlich treten leichte Schmierblutungen auf. Erst wenn der Tumor größer wird und mit Geschwürbildung zerfällt, kommt es zu fleischwasserfarbigem, süßlich riechendem Ausfluss. Im Frühstadium ist die vollständige Entfernung der Veränderung durch eine Konisation ausreichend. Im fortgeschrittenen Stadium werden die Entfernung der ganzen Gebärmutter mit umliegendem Gewebe und manchmal auch weiterer Organe notwendig.

In der weltweiten Todesursachenstatistik der gynäkologischen Malignome steht besonders das in das umgebende Gewebe hineinwuchernde (invasive) Zervixkarzinom damit auf Rang eins, mit einer Sterblichkeit (Letalität) von über 60 %. Jährlich erkranken in Deutschland etwa über 6000 Frauen neu an einem Zervixkarzinom, etwa 1800 sterben daran. Die 5-Jahres-Überlebenswahrscheinlichkeit der Patientinnen beträgt etwa 60 %.

Das Zervixkarzinom wird am häufigsten im Alter von 45 bis 55 Jahren diagnostiziert, doch Vorstufen können schon bei 20- bis 30-jährigen Patientinnen auftreten. Das mittlere Alter bei der Erstdiagnose des Zervixkarzinoms sank in den letzten 25 Jahren um 14 Jahre und liegt derzeit bei etwa 52 Jahren. In der Altersverteilung findet man einen Gipfel zwischen dem 35. und 54. Lebens jahr sowie einen weiteren Anstieg ab dem 65. Lebens jahr. 2003 zeigte die Erkrankungshäufigkeit eine veränderte Altersverteilung, weil die Diagnose deutlich häufiger bei Frauen in einem Alter zwischen 25 und 35 Jahren gestellt wurde als bei Frauen, die über 65 Jahre alt waren. Die Erkrankung kann auch in der Schwangerschaft auftreten. Die Inzidenz beträgt hier 1,2 pro 10.000 Schwangerschaften.

Man geht davon aus, dass ein großer Teil der Gebärmutterhalskarzinome von den humanen Papillomviren (HPV) verursacht wird. Eine Untersuchung zur Früherkennung ist der Pap-Test. Jedoch entwickeln sich nur bei 2 bis 8 Prozent der HPV-infizierten Frauen Zellveränderungen, die ein Vorstadium für eine Krebserkrankung darstellen, oder sogar anschließend ein Karzinom.

Die Diagnose eines Zervixkarzinoms kann nur durch histologische Untersuchung von Gewebestücken gestellt werden. Diese werden entweder durch eine gezielte Probenentnahme aus einem bei der Kolposkopie auffälligen Bereich am Muttermund, eine Konisation nach wiederholt auffälligem Pap-Test oder eine Ausschabung bei Verdacht auf eine im Gebärmutterhalskanal befindliche Veränderung gewonnen.

Bei gynäkologischen Malignomen, insbesondere Zervixkarzinom ist eine frühzeitige Diagnose für den weiteren Krankheitsverlauf und die Prognose entscheidend.

Es besteht deshalb ein Bedürfnis an indikationsspezifischen diagnostischen Vorrichtungen und Verfahren für gynäkologisches Malignom, insbesondere für Zervixkarzinom. Es ist die Aufgabe der vorliegenden Erfindung verbesserte Mittel zur Früherkennung und Therapiesteuerung bei gynäkologischem Malignom bereit zu stellen.

Die Erfindung betrifft eine Anordnung von Markersequenzen für gynäkologisches Malignom auf einem Träger zur Früherkennung, Diagnose, Prognose, Therapiesteuerung bei mit einer oder mehreren verschiedenen Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 (Klon Sequenzen, cDNA) und/oder kodiert durch Sequenzen SEQ ID. No. 490 - 978 (RNA Sequenzen) und/oder kodiert durch Teilsequenzen von SEQ ID. No. 1 - 978.

Gegenstand der Erfindung ist auch eine Anordnung von Markersequenzen für gynäkologisches Malignom auf einem Träger zur Analyse von Autoantikörperprofilen von gynäkologischem Malignom mit einer oder mehreren verschiedenen Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID. No. 1 - 978.

Im Rahmen dieser Erfindung wurde für die Proteine SEQ ID No. 979 bis 1467 und/oder Teilsequenzen dieser Proteine und/oder Proteine kodiert durch Sequenzen SEQ ID No. 1 - 489 und/oder Proteine kodiert durch Sequenzen SEQ ID. No. 490 - 978 und/oder kodiert durch Teilsequenzen von SEQ ID. No. 1 - 978 erstmals eine Verwendung für gynäkologisches Malignom gefunden und in der erfindungsgemäßen Anordnung umgesetzt. Die Erfindung stellt damit ein Panel von Markersequenzen für gynäkologisches Malignom zur Verfügung, die Rahmen einer individualisierten Diagnostik und Therapie verwendet werden können, um bei unterschiedlichen Patienten, Patientengruppen, Kohorten, Bevölkerungsgruppen, Varianten von gynäkologischem Malignom u.s.w. gezielt und individuell angepasst gynäkologisches Malignom zu diagnostizieren und die Therapie zu überwachen.

Gegenstand der Erfindung ist auch die Verwendung von einer oder mehreren Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID. No. 1 - 978 für Diagnose und Therapie von gynäkologischem Malignom, insbesondere zur Früherkennung von gynäkologischem Malignom, zur Diagnose von gynäkologischem Malignom, zur Prognose, beispielsweise des Risikos der Metastasenbildung, Therapiesteuerung, beispielsweise Vorhersage und Überwachung des Ansprechens auf ein Arzneimittel oder eine Therapie (Vorhersage der Sensitivität oder Resistenz), Nachsorge. Die Erfindung betrifft insbesondere auch den Nachweis und die Bestimmung der Menge von mindestens zwei verschiedenen Autoantikörpern bei einem Patienten wobei entsprechend mindestens zwei verschiedene erfindungsgemäße Markersequenzen für gynäkologisches Malignom (als Antigene) verwendet werden.

In bevorzugten Ausführungsformen der Erfindung umfasst die erfindungsgemäße Anordnung/Verwendung 2 oder 3, vorzugsweise 4 oder 5, besonders bevorzugt 7 oder 8 oder mehr unterschiedliche Markersequenzen für gynäkologisches Malignom. Die Anordnung/Verwendung kann 9 oder 10 oder mehr Markersequenzen für gynäkologisches Malignom umfassen, beispielsweise 10 bis 50, vorzugsweise 50 bis 100 oder mehr. Erfindungsgemäß umfasst sind auch Anordnungen/Verwendungen, die für Patienten individuell hergestellt werden, beispielsweise im Rahmen der individualisierten (personalisierten) Medizin. Gegenstand der Erfindung ist somit auch eine Anordnung/Verwendung wobei mindestens 2 bis 5 oder 10, vorzugsweise 30 bis 50 Markersequenzen für gynäkologisches Malignom oder 50 bis 100 oder mehr Markersequenzen für gynäkologisches Malignom an oder zu einem zu untersuchenden Patienten bestimmt werden.

Eine bevorzugte Ausführungsform der Erfindung betrifft eine Anordnung/Verwendung, dadurch gekennzeichnet, dass die Markersequenzen für gynäkologisches Malignom auf einem festen Träger aufgebracht werden, insbesondere einem Filter, einer Membran, einem magnetisches oder Fluorophor-markiertes Kügelchen, einem Silizium-Wafer, Glas, Metall, Kunststoff, einem Chip, einem massenspektrometrisches Target oder einer Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

Eine weitere Ausführungsform betrifft eine Anordnung/Verwendung, dadurch gekennzeichnet, dass die Markersequenzen für gynäkologisches Malignom als Klone vorliegen.

Daher betrifft die Erfindung die Verwendung von Markersequenzen für gynäkologisches Malignom zur Diagnose von gynäkologischem Malignom, wobei mindestens eine Markersequenz für gynäkologisches Malignom einer DNA, insbesondere cDNA ausgewählt aus der Gruppe SEQ ID No. 1-489 oder RNA ausgewählt aus der Gruppe 490 - 978 oder einer Teilsequenz oder eines Fragments davon an oder zu einem zu untersuchenden Patienten bestimmt wird.

Die Bereitstellung von Markersequenzen für gynäkologisches Malignom (nachstehend auch erfindungsgemäße Markersequenzen genannt) erlaubt eine sichere Diagnose und Stratifizierung von Patienten mit gynäkologischem Malignom insbesondere mittels eines Proteinbiochips.

Die erfindungsgemäßen Markersequenzen für gynäkologisches Malignom konnten mittels differentiellem Screenen von Proben und zwar gesunder Probanden mit Patientenproben mit gynäkologischem Malignom identifiziert werden. Hierbei konnten diese erfindungsgemäßen Markersequenzen erstmals mittels Proteinbiochips (siehe Beispiele) identifiziert werden.

Gegenstand der Erfindung ist auch ein Verfahren zur Identifizierung von Markersequenzen für gynäkologisches Malignom umfassend die Schritte
a) Bereitstellung von Markersequenzen auf einem Array,
b) Identifizierung von Markersequenzkandidaten für gynäkologisches Malignom durch vergleichende Analyse der Signale, die bei Kontakt der Markersequenzen mit
   Körperflüssigkeit oder Gewebeauszug eines Patienten mit gynäkologischem Malignom und
   Körperflüssigkeit oder Gewebeauszug eines Patienten ohne gynäkologisches Malignom gemessen werden,
c) Charakterisierung der Markersequenzkandidaten für gynäkologisches Malignom mit Hilfe eines Proteinbiochips,
d) Auswahl von Markersequenzkandidaten für gynäkologisches Malignom, die ein unterschiedliches Signal bei Patienten mit gynäkologischem Malignom und Patienten ohne gynäkologisches Malignom liefern (Markersequenzen für gynäkologisches Malignom).

Der Begriff" gynäkologisches Malignom" umfasst eine Gruppe von Erkrankungen, die Vorstufen zu gynäkologischem Malignom sein können und deren Etablierung als gynäkologischem Malignom. Umfasst sind maligne Erkrankungen des Genitaltraktes bei Frauen, insbesondere maligne Erkrankungen des Gebärmutterhalses wie Zervixkarzinom, insbesondere invasives Zervixkarzinom (Definition z.B. nach Pschyrembel, de Gruyter, 263. Auflage (2012), Berlin).

In einer weiteren Ausführungsform der Erfindung (z.B. Anordnung, Verwendung) können die erfindungsgemäßen Markersequenzen für gynäkologisches Malignom ebenfalls mit bekannten Biomarkern für diese Indikation kombiniert, ergänzt oder erweitert werden. Dabei sind jedoch mindestens 50 %, vorzugsweise 60 %, besonders bevorzugt 70 % oder mehr erfindungsgemäße Markersequenzen vertreten, beispielsweise in der erfindungsgemäßen Anordnung. In besonders bevorzugten Ausführungsformen der Erfindung, insbesondere der erfindungsgemäßen Anordnung, dem erfindungsgemäßen Assay und Proteinbiochip sowie der erfindungsgemäßen Verwendung sind mindestens 75 %, vorzugsweise 80 % oder 85 %, besonders bevorzugt 90 % oder 95 % erfindungsgemäße Markersequenzen vertreten.

In einer bevorzugten Ausführungsform erfolgt die Bestimmung der Markersequenzen für gynäkologisches Malignom außerhalb des menschlichen Körpers und die Bestimmung erfolgt in einer ex vivo / in vitro Diagnose.

Gegenstand der Erfindung ist auch ein Assay oder Proteinbiochip umfassend eine erfindungsgemäße Anordnung/Verwendung. Die Erfindung betrifft eine diagnostische Vorrichtung und/oder einen Assay, insbesondere einen Proteinbiochip der für gynäkologisches Malignom eine Früherkennung, Diagnose, Prognose, Stratifizierung und/oder Untersuchung erlaubt.

Die Erfindung betrifft auch die Verwendung einer erfindungsgemäßen Anordnung oder eines erfindungsgemäßen Assays oder Proteinbiochips zur Analyse von Autoantikörperprofilen von Patienten, insbesondere zur quantitativen Analyse und/oder zur Überwachung von Veränderungen von Autoantikörperprofilen von Patienten.

Gegenstand der Erfindung ist auch ein Diagnostikum (Test Kit) zur Früherkennung und/oder Diagnose von gynäkologischem Malignom und/oder Prognose und/oder Vorhersage des Risikos der Metastasenbildung bei gynäkologischem Malignom umfassend eine erfindungsgemäße Anordnung vorzugsweise auf einem Träger oder einen erfindungsgemäßen Assay oder Proteinbiochip und gegebenenfalls weitere Zusatz- und Hilfsstoffe.

Gegenstand der Erfindung ist auch ein Diagnostikum (Test Kit) zur Therapieüberwachung und/oder Nachsorge bei gynäkologischem Malignom umfassend eine erfindungsgemäße Anordnung oder einen erfindungsgemäßen Assay oder Proteinbiochip und gegebenenfalls weitere Zusatz- und Hilfsstoffe.

In einer weiteren Ausführungsform der Erfindung betrifft die Erfindung die Verwendung von Markersequenzen für gynäkologisches Malignom als Diagnostika, wobei mindestens eine Markersequenz einer cDNA ausgewählt aus der Gruppe SEQ ID No. 1-489 (Klon Sequenzen) oder SEQ ID No. 490 - 978 (RNA) oder jeweils ein durch SEQ ID No. 1-978 kodiertes Protein oder jeweils eine Teilsequenz oder Fragment davon ist.

Gegenstand der Erfindung ist auch ein Verfahren zur Früherkennung und Diagnose von gynäkologischem Malignom wobei
a.) eine oder mehrere Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID No. 1 - 978 auf einem Träger aufgebracht werden und
b.) mit Körperflüssigkeit oder Gewebeauszug eines Patienten in Kontakt gebracht werden und
c.) der Nachweis einer Wechselwirkung der Körperflüssigkeit oder Gewebeauszug mit den Markersequenzen für gynäkologisches Malignom aus a.) erfolgt.

Ferner betrifft die Erfindung ein Verfahren zur Früherkennung und Diagnose von gynäkologischem Malignom wobei
a.) mindestens eine Markersequenz für gynäkologisches Malignom einer cDNA ausgewählt aus der Gruppe SEQ ID No. 1-489 (Klon Sequenzen) oder SEQ ID No. 490 - 978 (RNA) oder eine Teilsequenz von SEQ ID. No. 1-978 auf einen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

Eine besondere Ausführungsform der Erfindung betrifft Verfahren zur Früherkennung und Diagnose von gynäkologischem Malignom, wobei die Wechselwirkung nach c.) ein Autoantikörperprofil assoziiert mit gynäkologischem Malignom des Patienten oder einer Kohorte oder einer Bevölkerungsgruppe oder eines bestimmten Krankheitsverlaufs (Prognose) oder eines bestimmten Ansprechens auf eine Therapie/Arzneimittel abbildet.

In einem Verfahren zur Diagnose und/oder in einem Diagnostikum werden ein oder mehrere Markersequenzen für gynäkologisches Malignom verwendet. In einer bevorzugten Ausführungsform werden mindestens 2, beispielsweise 3, 4, 5, 6, 7, 8, 9, 10, vorzugsweise 15 bis 20 Markersequenzen oder 30 bis 50 oder 100 oder mehr Markersequenzen für gynäkologisches Malignom zusammen oder in Kombination verwendet, beispielsweise direkt nacheinander oder parallel.

Der Nachweis einer Wechselwirkung der Körperflüssigkeit oder des Gewebeauszugs mit der oder den Markersequenzen für gynäkologisches Malignom kann beispielsweise durch eine Sonde, insbesondere durch einen Antikörper erfolgen.

Gegenstand der Erfindung ist auch ein Verfahren zum Stratifizieren, insbesondere zur Risikostratifizierung, oder zur Therapiesteuerung eines Patienten mit gynäkologischem Malignom, wobei mit Hilfe einer oder mehrerer Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID No. 1 - 978 das Autoantikörperprofil assoziiert mit gynäkologischem Malignom eines Patienten bestimmt und gegebenenfalls überwacht wird.

Eine besondere Ausführungsform der Erfindung betrifft das Verfahren, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

Ferner betrifft die Erfindung ein Verfahren zum Stratifizieren, insbesondere zur Risikostratifizierung oder Therapiesteuerung eines Patienten mit gynäkologischem Malignom, wobei mindestens eine Markersequenz einer Nuleinsäure, beispielsweise cDNA ausgewählt aus der Gruppe SEQ ID No. 1-489 (Klon Sequenzen) oder SEQ ID No. 490 - 978 (RNA) oder jeweils ein dadurch kodiertes Protein oder jeweils eine Teilsequenz von SEQ ID No. 1 - 978 an einem zu untersuchenden Patienten bestimmt wird.

Ferner betrifft die Erfindung ein Verfahren zum Stratifizieren, insbesondere zur Risikostratifizierung und / oder Therapiesteuerung eines Patienten mit gynäkologischem Malignom, wobei mindestens eine Markersequenz einer DNA, cDNA ausgewählt aus der Gruppe SEQ ID No. 1-489 (Klon Sequenzen) oder SEQ ID No. 490 - 978 (RNA bzw. DNA) oder jeweils eine Teilsequenz davon verwendet wird um Autoantikörper und/oder Autoantikörperprofile assoziiert mit gynäkologischem Malignom bei dem Patienten nachzuweisen, zu identifizieren, zu überwachen.

Autoantikörperprofile umfassen die Menge an einem oder mehreren Autoantikörpern deren Vorkommen/Expression mit der Entstehung und/oder Etablierung von gynäkologischem Malignom einhergehen.

Ferner umfasst ist die Stratifizierung der Patienten mit gynäkologischem Malignom in neue oder etablierte Subgruppen der gynäkologischem Malignom Patienten, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung von gynäkologischem Malignom mittels der erfindungsgemäßen Markersequenzen für gynäkologisches Malignom sowie die Zuordnung der Patienten zu gynäkologischem Malignom. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von gynäkologischem Malignom mittels der erfindungsgemäßen Markersequenzen für gynäkologisches Malignom sowie die Prognose von gynäkologischem Malignom, insbesondere die Vorhersage des Risikos der Metastasenbildung.

"Stratifizieren (auch: Stratifikation) oder Therapiesteuerung" im Sinne dieser Erfindung bedeutet, dass beispielsweise die erfindungsgemäßen Verfahren Entscheidungen zur Behandlung und Therapie des Patienten erlauben, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

"Prognose" bedeutet die Vorhersage des Krankheitsverlaufs, beispielsweise die Vorhersage des Rezidiv-freien Überlebens, des Gesamtüberlebens, des Risikos der Metastasenbildung.

Im Rahmen dieser Erfindung wird unter "Patient" ein beliebiger Proband - Mensch oder Säugetier - verstanden, mit der Maßgabe, dass der Proband auf gynäkologisches Malignom untersucht wird.

Der Begriff "Markersequenz für gynäkologisches Malignom" im Sinne dieser Erfindung bedeutet, dass die Nukleinsäure, beispielsweise DNA, insbesondere cDNA oder RNA oder die Aminosäuresequenz oder das jeweils daraus erhältliche Polypeptid oder Protein oder die durch die Nukleinsäuren kodierten Aminosäuren (Protein, Peptid) signifikant (spezifisch) für gynäkologisches Malignom sind.

Markersequenzen für gynäkologisches Malignom können Nukleinsäuresequenzen und Aminosäuresequenzen sein, wobei auch Modifikationen umfasst sind.

"für gynäkologisches Malignom" bedeutet, dass beispielsweise die cDNA oder das jeweils daraus erhältliche Polypeptid oder Protein eine Wechselwirkung mit Substanzen aus der Körperflüssigkeit oder Gewebeauszug eines Patienten mit gynäkologischem Malignom aufweisen (z.B. Antigen (Epitop) / Antikörper (Paratop) Wechselwirkung). Diese Substanzen aus der Körperflüssigkeit oder Gewebeauszug treten entweder nur oder zumindest verstärkt bei gynäkologischem Malignom auf beziehungsweise werden exprimiert, wohingegen diese Substanzen bei Patienten ohne gynäkologischem Malignom nicht oder zumindest in geringerem Maße (geringere Menge, geringere Konzentration) vorhanden sind. Markersequenzen für gynäkologisches Malignom können sich andererseits auch dadurch auszeichnen, dass sie eine Wechselwirkung mit Substanzen aus der Körperflüssigkeit oder Gewebeauszug von Patienten mit gynäkologischem Malignom eingehen, weil diese Substanzen nicht mehr oder zumindest in deutlich geringerer Menge/Konzentration bei gynäkologischem Malignom auftreten beziehungsweise exprimiert werden, wohingegen diese Substanzen bei Patienten ohne gynäkologischem Malignom vorhanden oder zumindest in deutlich höherem Maße vorhanden sind. Markersequenzen für gynäkologisches Malignom können auch in gesunden Probanden vorhanden sein, jedoch verändert sich ihre Menge (Konzentration) beispielsweise bei der Entstehung, Etablierung und Therapie von gynäkologischem Malignom. Die Markersequenzen für gynäkologisches Malignom sind deshalb Biomarker für gynäkologisches Malignom. Die Markersequenzen für gynäkologisches Malignom können auf diese Weise ein Profil von Substanzen aus Körperflüssigkeit und Gewebeauszug abbilden, beispielsweise ein Autoantikörperprofil für gynäkologisches Malignom.

"Autoantikörperprofil für gynäkologisches Malignom" umfasst somit einerseits die Zusammensetzung (ein oder mehrerer Autoantikörper) und anderseits die Menge/Konzentration einzelner Autoantikörper.

In einer besonders bevorzugten Ausführungsform der Erfindung ist die Markersequenz für gynäkologisches Malignom ein Antigen oder ein Teil eines Antigens oder kodiert für ein Antigen oder für einen Teil eines Antigens.

In einer besonders bevorzugten Ausführungsform erkennt/bindet die Markersequenz für gynäkologisches Malignom an Autoantikörper, die im Verlauf der Entstehung, Etablierung und Therapie von gynäkologischem Malignom (verstärkt) vorhanden sind oder in geringerem Maße (oder nicht mehr) vorhanden sind (nachfolgend "Autoantikörper für gynäkologisches Malignom"). Autoantikörper werden vom Körper gegen körpereigene Antigene, die beispielsweise bei gynäkologischem Malignom entstehen, gebildet. Autoantikörper werden von Körper gegen unterschiedliche Substanzen und Pathogenen gebildet. Im Rahmen der vorliegenden Erfindung werden insbesondere die Autoantikörper für gynäkologisches Malignom detektiert, die beim Auftreten und im Laufe der Entstehung von gynäkologischem Malignom gebildet werden und/oder in ihrer Expression hoch- beziehungsweise herunterreguliert werden. Autoantikörper assoziiert mit gynäkologischem Malignom können mit Hilfe der erfindungsgemäßen Verfahren und Markersequenzen für gynäkologisches Malignom nachgewiesen werden und somit als Indikation für gynäkologisches Malignom dienen. Der Nachweis und die Überwachung der Menge an Autoantikörpern für gynäkologisches Malignom im Patienten kann zur Früherkennung, Diagnose und/oder Therapieüberwachung/Therapiesteuerung verwendet werden. Diese Autoantikörperprofile assoziiert mit gynäkologischem Malignom können bereits bei Verwendung von einer Markersequenz für gynäkologisches Malignom ausreichend charakterisiert werden. In anderen Fällen werden zwei oder mehr Markersequenzen für gynäkologisches Malignom notwendig sein, um ein Autoantikörperprofil assoziiert mit gynäkologischem Malignom abzubilden.

In bevorzugten Ausführungsformen der Erfindung können die Autoantikörper assoziiert mit gynäkologischem Malignom mit Markersequenzen für gynäkologisches Malignom nachgewiesen werden, die sich von einem anderen Individuum ableiten, weil sie beispielsweise aus einer kommerziellen cDNA-Bank stammen. Bevorzugte Ausführungsformen der Erfindung betreffen die Markersequenzen für gynäkologisches Malignom SEQ ID No. 1 - 489, SEQ ID. No. 490 - 978 und/oder Teilsequenzen von SEQ ID No. 1 - 978, und Sequenzen, die für die Proteine SEQ ID No. 979 bis 1467 und/oder Teilsequenzen dieser Proteine kodieren.

In anderen bevorzugten Ausführungsformen der Erfindung können die Autoantikörper für gynäkologisches Malignom mit Markersequenzen für gynäkologisches Malignom nachgewiesen werden, die sich von dem gleichen Individuum ableiten (Autoantigen), weil sie beispielsweise aus einer eigens für den Patienten oder eine Gruppe von Patienten (beispielsweise im Rahmen der individualisierten Medizin) hergestellten cDNA-Bank stammen. Beispielsweise werden dann Homologe der genannten Markersequenzen für gynäkologisches Malignom SEQ ID. No. 1 - 1467 oder Teilsequenzen davon verwendet.

Autoantikörper können bereits viele Jahre vor Auftreten der ersten Krankheitssymptome vom Patienten gebildet werden. Damit wäre eine Früherkennung, Diagnose und auch Prognose und (vorbeugende) Behandlung bereits Jahre vor dem sichtbaren Krankheitsausbruch möglich. Die erfindungsgemäßen Vorrichtungen und Mittel (Anordnung, Array, Proteinbiochip, Diagnostikum, Test Kit) und Verfahren ermöglichen somit ein im Vergleich zu bekannten Verfahren sehr frühes Eingreifen, was die Prognose und Überlebensraten deutlich verbessert. Da sich die Autoantikörperprofile assoziiert mit gynäkologischem Malignom während der Etablierung und Behandlung/Therapie von gynäkologischem Malignom verändern, ermöglicht die Erfindung auch den Nachweis und die Überwachung von gynäkologischem Malignom in jedem Stadium der Entstehung und Behandlung sowie die Überwachung im Rahmen der Nachsorge bei gynäkologischem Malignom. Die erfindungsgemäßen Mittel erlauben auch eine einfache Handhabung zu Hause durch den Patienten und die kostengünstige routinemäßige Vorsorge zur Früherkennung.

Insbesondere durch die Verwendung von Antigenen als spezifische Markersequenz für gynäkologisches Malignom, die sich von bereits bekannten Sequenzen, z.B. aus kommerziellen cDNA Banken, ableiten, können Probanden getestet und gegebenenfalls vorhandene Autoantikörper assoziiert mit gynäkologischem Malignom in diesen Probanden nachgewiesen werden, auch wenn bei diesen Probanden die entsprechenden Autoantigene (noch) nicht bekannt sind.

Verschiedene Patienten können unterschiedliche Autoantikörperprofile assoziiert mit gynäkologischem Malignom aufweisen, beispielsweise unterscheiden sich verschiedene Kohorten oder Bevölkerungsgruppen. Jeder Patient kann dabei ein oder mehrere verschiedene Autoantikörper assoziiert mit gynäkologischem Malignom im Laufe der Entstehung von gynäkologischem Malignom und des Fortschreitens der Erkrankung des gynäkologischen Malignoms, d.h. ebenfalls verschiedene Autoantikörperprofile, bilden. Außerdem kann sich die Zusammensetzung und/oder die Menge der gebildeten Autoantikörper assoziiert mit gynäkologischem Malignom im Laufe der gynäkologischem Malignom Entstehung und des Fortschreitens der Krankheit verändern, so dass eine quantitative Auswertung notwendig wird. Auch die Therapie/Behandlung von gynäkologischem Malignom führt zu Veränderungen in der Zusammensetzung und/oder der Menge an Autoantikörpern assoziiert mit gynäkologischem Malignom. Die große Auswahl an erfindungsgemäßen Markersequenzen für gynäkologisches Malignom ermöglichen die individuelle Zusammenstellung von Markersequenzen für gynäkologisches Malignom in einer Anordnung für einzelne Patienten, Gruppen von Patienten, bestimmte Kohorten, Bevölkerungsgruppen und so weiter. Im Einzelfall kann deshalb die Verwendung einer Markersequenz für gynäkologisches Malignom ausreichen, während in anderen Fällen mindestens zwei oder mehr Markersequenzen für gynäkologisches Malignom zusammen oder in Kombination verwendet werden müssen um ein aussagekräftiges Autoantikörperprofil zu erstellen.

Der Nachweis von Autoantikörpern assoziiert mit gynäkologischem Malignom beispielsweise im Serum/Plasma hat gegenüber anderen Biomarkern den Vorteil einer hohen Stabilität und Lagerfähigkeit und einer guten Nachweisbarkeit. Auch unterliegt die Anwesenheit von Autoantikörpern keinem circardianen Rhythmus, so dass die Probennahme unabhängig von Tageszeit, Nahrungsaufnahme und dergleichen ist.

Daneben können Autoantikörper assoziiert mit gynäkologischem Malignom mit Hilfe der korrespondierenden Antigene/Autoantigene in bekannten Assays wie z.B. ELISA oder Western-Blot nachgewiesen werden und auf dieses die Ergebnisse überprüft werden.

Im Sinne der Erfindung bedeutet "wobei mindestens eine Markersequenz für gynäkologisches Malignom ausgewählt wird", dass eine Wechselwirkung nachgewiesen wird. Eine solche Wechselwirkung ist z.B. eine Bindung, insbesondere eine bindende Substanz an mindestens eine Markersequenz für gynäkologisches Malignom oder im Fall, dass die Markersequenz für gynäkologisches Malignom eine Nukleinsäure, beispielsweise eine cDNA ist, die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Die Wechselwirkung zwischen der Körperflüssigkeit oder Gewebeauszuges eines Patienten und den Markersequenzen für gynäkologisches Malignom ist vorzugsweise eine Protein-Protein Wechselwirkung.

Solche Substanzen, beispielsweise Antigene, Autoantigene, Autoantikörper assoziiert mit gynäkologischem Malignom, sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges, beispielsweise aus Tumorgewebe des Patienten. Die Erfindung betrifft insbesondere die Verwendung dieser Körperflüssigkeiten und Gewebeauszüge für Früherkennung, Diagnose, Prognose, Therapiesteuerung und Nachsorge.

In einer weiteren Ausführungsform der Erfindung können jedoch die Markersequenzen für gynäkologisches Malignom bzw. die von diesen Markersequenzen erkannten Substanzen, beispielsweise Autoantikörper assoziiert mit gynäkologischem Malignom, in einer signifikant höheren oder niedrigeren Expressionsrate oder Konzentration vorliegen, was auf gynäkologischem Malignom hinweist. Hierbei werden mittels Proteomics oder Nukleinsäureblots die relativen Expressionsraten krank / gesund der erfindungsgemäßen Markersequenzen für gynäkologisches Malignom bzw. die von diesen Markersequenzen erkannten Substanzen bestimmt.

Die Markersequenzen für gynäkologisches Malignom verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen für gynäkologisches Malignom sind Gegenstand der Tabelle A (RNA), Tabelle B (Proteinsequenzen) und des Sequenzprotokolls und können durch den jeweilig zitierten Datenbankeintrag (auch mittels Internet: http://www.ncbi.nlm.nih.gov/) eindeutig identifiziert werden (mittels Accession No.), siehe ebenfalls das zugehörige Sequenzprotokoll. Die Klonsequenzen (cDNA) sind nur im Sequenzprotokoll angegeben.

Daher umfasst die Erfindung ebenfalls die Volllängesequenzen der erfindungsgemäßen Markersequenzen für gynäkologisches Malignom und zwar wie über den bekannten Datenbankeintrag gemäß Tabellen A, B, sowie im Sequenzprotokoll definiert, nachstehend SEQ 1 - 1467 genannt.

Weiterhin umfasst sind daher ebenfalls analoge Ausführungsformen von SEQ 1 -1467 zu den Markersequenzen für gynäkologisches Malignom SEQ 1-1467, wie z.B. in den Ansprüchen dargelegt, da die erfindungsgemäßen SEQ 1-1467 wiederum Teilsequenzen, zumindest mit hoher Homologie, darstellen. Die Markersequenzen für gynäkologisches Malignom SEQ 1-1467 sind jedoch erfindungsgemäß bevorzugt.

Erfindungsgemäß umfassen die Markersequenzen auch solche Modifikationen der Nukleinsäuresequenz, insbesondere cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

Die Erfindung betrifft auch Homologe der Markersequenzen für gynäkologisches Malignom und Teilsequenzen, beispielsweise Fragmente von Markersequenzen für gynäkologisches Malignom. Homologe sind beispielsweise Nukleinsäure- und/oder Proteinsequenzen, insbesondere Homologe von SEQ ID No. 1 - 1467, die eine Identität mit den Markersequenzen für gynäkologisches Malignom von mindestens 70 % oder 80 %, vorzugsweise 90 % oder 95 %, besonders bevorzugt 96 % oder 97 % oder mehr, beispielsweise 98 % oder 99 % aufweisen. In einer besonders bevorzugten Ausführungsform der Erfindung beträgt für den Fall, dass es sich bei den Markersequenzen für gynäkologisches Malignom um Antigene handelt, die Homologie im Sequenzbereich in dem die Antigen-Antikörper- beziehungsweise Antigen-Autoantikörper-Wechselwirkung stattfindet mindestens 95 %, vorzugsweise mindestens 97 %, besonders bevorzugt mindestens 99 %.

Gegenstand der Erfindung sind auch Teilsequenzen der Markersequenzen für gynäkologisches Malignom. Teilsequenzen umfassen auch Fragmente der erfindungsgemäßen Markersequenzen, Teilsequenzen sind solche Nukleinsäuren oder Proteine/Peptide, die gegenüber der vollständigen Nukleinsäure oder dem vollständigen Protein/Peptid verkürzt sind. Die Deletion kann sich dabei an dem oder den Ende und/oder innerhalb der Sequenz befinden. Umfasst sind beispielsweise Teilsequenzen und/oder Fragmente die 50 bis 100 Nukleotide, 70-120 Nukleotide einer vollständigen Sequenz aufweisen, beispielsweise von SEQ 1-1467. Homologe von Teilsequenzen und Fragmenten sind ebenfalls erfindungsgemäß umfasst. In einer besonderen Ausführungsform sind die Markersequenzen für gynäkologisches Malignom gegenüber den Sequenzen 1 - 1467 so weit verkürzt, dass sie nur noch aus der/den Bindungsstellen für den betreffenden Autoantikörper assoziiert mit gynäkologischem Malignom bestehen. Erfindungsgemäß umfasst sind auch Markersequenzen für gynäkologisches Malignom, die sich von den Sequenzen SEQ ID No. 1 - 1467 dadurch unterscheiden, dass sie ein oder mehrere Insertionen beinhalten, wobei die Insertionen beispielsweise 1 bis 100 oder mehr Nukleotide/Aminosäuren, vorzugsweise 5 bis 50, besonders bevorzugt 10 bis 20 Nukleotide/Aminosäuren lang sind und die Sequenzen aber ansonsten identisch oder homolog zu den Sequenzen 1 - 1467 sind.

In einer weiteren Ausführungsform kann die jeweilige Markersequenz für gynäkologisches Malignom in unterschiedlichen Mengen in einem oder mehreren Bereichen auf dem Träger repräsentiert sein. Dies erlaubt eine Variation der Sensitivität. Die Bereiche können jeweils eine Gesamtheit von Markersequenzen für gynäkologisches Malignom aufweisen, d.h. eine genügende Zahl an verschiedenen Markersequenzen für gynäkologisches Malignom, insbesondere 2, 3, 4, 5, 6, 7, 8, 9 oder 10 oder mehr verschiedene und gegebenenfalls weitere Nukleinsäuren und/oder Proteinen, insbesondere Biomarker.

In einer besonders bevorzugten Ausführungsform der Erfindung sind mindestens 96 bis 25.000 (numerisch) oder mehr verschiedene oder gleiche Markersequenzen für gynäkologisches Malignom und gegebenenfalls weitere Nukleinsäuren und/oder Proteinen, insbesondere Biomarker auf dem Träger repräsentiert. Weiterhin bevorzugt sind mehr als 2.500, besonders bevorzugt 10.000 oder mehr verschiedene oder gleiche Markersequenzen für gynäkologisches Malignom und gegebenenfalls weitere Nukleinsäuren und/oder Proteine, insbesondere Biomarker auf dem Träger repräsentiert.

Im Rahmen dieser Erfindung bedeutet "Anordnung" synonym "Array" und sofern dieser "Array" zur Identifizierung von Substanzen an Markersequenzen für gynäkologisches Malignom verwendet wird, ist hierunter vorzugsweise ein "Assay" oder eine diagnostische Vorrichtung zu verstehen. In einer bevorzugten Ausführungsform ist die Anordnung derart gestaltet, dass die auf der Anordnung repräsentierten Markersequenzen für gynäkologisches Malignom in Form eines Gitters auf einem Träger vorliegen. Ferner sind solche Anordnungen bevorzugt, die eine hochdichte (high-density) Anordnung von Markersequenzen für gynäkologisches Malignom, beispielsweise Proteinbindern erlauben. Vorzugsweise werden die Markersequenzen für gynäkologisches Malignom gespottet. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren.

Ein "Proteinbiochip" im Sinne dieser Erfindung ist die systematische Anordnung von Markersequenzen für gynäkologisches Malignom auf einem festen Träger, wobei die Markersequenzen für gynäkologisches Malignom Proteine oder Peptide oder Teile davon sind.

Die Markersequenzen für gynäkologisches Malignom der Anordnung sind auf einen festen Träger fixiert, vorzugsweise jedoch gespottet oder immobilisiert gar aufgedruckt, d.h. reproduzierbar aufgebracht. Ein oder mehrere Markersequenzen für gynäkologisches Malignom können mehrfach in der Gesamtheit aller Markersequenzen für gynäkologisches Malignom präsent sein und in unterschiedlichen Mengen bezogen auf einen Spot vorliegen. Ferner können die Markersequenzen für gynäkologisches Malignom auf dem festen Träger standardisiert sein (z.B. mittels serieller Verdünnungsreihen von z.B. Humanglobulinen als interne Kalibratoren zur Datennormalisierung und quantitativen Auswertung).

In einer weiteren Ausführungsform liegen die Markersequenzen für gynäkologisches Malignom als Klone vor. Solche Klone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Klone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe, beispielsweise aus Gewebe aus Eierstock und Gebärmutter. Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden.

Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Klone ebenfalls nicht abschließend solche sein wie transformierte Bakterien, rekombinante Phagen oder transformierte Zellen von Säugern, Insekten, Pilzen, Hefen oder Pflanzen.

Die Klone werden auf einen festen Träger fixiert, gespottet oder immobilisiert. Daher betrifft die Erfindung eine Anordnung/Verwendung, wobei die Markersequenzen für gynäkologisches Malignom als Klone vorliegen.

Zusätzlich können die Markersequenzen für gynäkologisches Malignom in der jeweiligen Form in Form eines Fusionsproteins vorliegen, welches beispielsweise mindestens ein Affinitätsepitop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, Calmodulin-bindendes Protein, Glutathione S-transferase oder lacZ enthalten.

In einer weiteren bevorzugten Ausführungsform der erfindungsgemäßen Anordnung/Verwendung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz (beispielsweise auch Hit, Leitsubstanz, Kandidat, Wirkstoff) für gynäkologisches Malignom, dadurch gekennzeichnet, dass eine erfindungsgemäße Anordnung oder Assay mit
a.) mindestens einer zu untersuchenden Substanz in Kontakt gebracht wird und
b.) ein Bindungserfolg nachgewiesen wird.

Die zu untersuchende Substanz kann ein beliebiges natives oder nicht-natives Biomolekül, ein (synthetisches) chemisches Molekül, ein Naturstoff, eine Mischung oder eine Substanzbibliothek sein.

Nachdem die zu untersuchende Substanz eine Markersequenz für gynäkologisches Malignom kontaktiert hat, erfolgt die Auswertung des Bindungserfolges, beispielsweise unter Verwendung mit handelsüblicher Image-Analyse Software (GenePix Pro (Axon Laboratories), Aida (Raytest), ScanArray (Packard Bioscience).

Die Visualisierung erfindungsgemäßer Bindungen, Bindungserfolge, Wechselwirkungen wie Protein-Protein-Wechselwirkungen (z.B. Protein an Markersequenz für gynäkologisches Malignom, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für gynäkologischem Malignom entwickelt und erhältlich durch den Einsatz einer erfindungsgemäßen Markersequenz, einer erfindungsgemäßen Anordnung, einer erfindungsgemäßen Verwendung, eines erfindungsgemäßen Assays.

Daher betrifft die Erfindung in einer weiteren Ausführungsform ein Target zur Behandlung und Therapie von gynäkologischem Malignom ausgewählt aus der Gruppe der Proteine SEQ ID No. 979 bis 1467, Teilsequenzen dieser Proteine und den für diese Proteine kodierenden Sequenzen, den Sequenzen SEQ ID No. 1 - 489 und SEQ ID. No. 490 - 978 und Teilsequenzen von SEQ ID No 1 - 978 sowie der von SEQ ID No. 1 - 978 kodierten Proteine.

Gegenstand der Erfindung ist auch die Verwendung einer Markersequenz für gynäkologisches Malignom ausgewählt aus der Gruppe der Proteine SEQ ID No. 979 bis 1467, Teilsequenzen dieser Proteine und den für diese Proteine kodierenden Sequenzen, den Sequenzen SEQ ID No. 1 - 489 und SEQ ID. No. 490 - 978 und Teilsequenzen von SEQ ID No 1 - 978 sowie der von SEQ ID No. 1 - 978 kodierten Proteine als Affinitätsmaterial zur Durchführung einer Apherese oder Blutwäsche für Patienten mit gynäkologischem Malignom. Die Erfindung betrifft somit die Verwendung der erfindungsgemäßen Markersequenzen, vorzugsweise in Form einer Anordnung, als Affinitätsmaterial zur Durchführung einer Blutwäsche im weiteren Sinne, wobei Substanzen aus Körperflüssigkeiten eines Patienten mit gynäkologischem Malignom, wie Blut oder Plasma, an die erfindungsgemäßen Markersequenzen binden und folglich der Körperflüssigkeit selektiv entzogen werden können.

Die nachfolgenden Beispiele dienen der Erläuterung der Erfindung ohne die Erfindung jedoch auf die Beispiele einzuschränken.

Die Durchführung der Beispiele erfolgt unter Verwendung der UNIarray Technologieplattform auf Basis quantitativer Analysen der Autoantikörperprofile im Serum von Patientinnen mit gynäkologischem Malignom. Dadurch sollen systematisch Antigene assoziiert mit gynäkologischem Malignom und Autoantigene assoziiert mit gynäkologischem Malignom (sogenannte Biomarker) identifiziert werden, die eine Früherkennung von gynäkologischem Malignom ermöglichen und/oder auf eine bestimmte Verlaufsform hindeuten (prognostische Relevanz).

### Beispiel 1

Zunächst erfolgt die Identifizierung von Kandidaten für Markersequenzen für gynäkologisches Malignom.

In der ersten Phase werden dazu 50 Serumproben von Patientinnen mit gynäkologischem Malignom auf einem MACROarray (umfasst etwa 10.000 unterschiedliche, rekombinante, humane Proteine) untersucht. Dabei werden Kandidaten für Markersequenzen für gynäkologisches Malignom identifiziert.

### Beispiel 2

In der anschließenden Testphase werden diese Kandidaten für Markersequenzen für gynäkologisches Malignom an Serumproben von 100 Patientinnen mit gynäkologischem Malignom und 100 Patientinnen mit benignen Veränderungen bzw. 100 gesunden Kontroll-Patientinnen vergleichend analysiert und charakterisiert. Dadurch diese vergleichende Analyse werden vorwiegend Markersequenzen identifiziert, die mit Autoantikörpern assoziiert mit gynäkologischem Malignom Wechselwirken.

### Beispiel 3:

Die Auswahl besonders signifikanter Biomarker ( Markersequenzen für gynäkologisches Malignom) erfolgt mittels bioinformatischer Analyse. Die Kandidaten für Markersequenzen für gynäkologisches Malignom werden dahingehend ausgewertet, ob sie zwischen verschiedenen Probanden (z.B. gesund/nicht gesund)/Patientengruppen (z.B. niedriges/hohes Risiko der Metastasenbildung)/Kohorten (z.B. bestimmte Vorgeschichte) diskriminieren.

Dazu werden die Markersequenzkandidaten werden auf einen Biochip aufgebracht und validiert. Die Datenauswertung erfolgt über statistische Analysen, beispielsweise Schwellenwertanalyse, Support Vector Machine Alogrithm (SVM). Der Probenverbrauch für die Validierung beträgt nur 50 µl / Probe. In einem ersten Ansatz werden auf diese Weise Kohorten der Kategorie I und II ausgewählt.

Der erhaltene Biochip ist für gynäkologisches Malignom spezifisch. Dieser Biochip umfasst ein oder mehrere Markersequenzen für gynäkologisches Malignom und erkennt Autoantikörper assoziiert mit gynäkologischem Malignom.

Kohorte I: Klinischer Befund: gynäkologisches Malignompositive Gruppe (CASE-Gruppe; verifiziert über histopathologischen Befund der Biopsie).

Kohorte II: Klinischer Befund: gynäkologisches Malignomnegative Gruppe (Kontroll-Gruppe), Alters-gematched.

Patientinnen werden ausgewählt nach Einschluß- und Ausschlusskriterien

Einschlußkriterien
- Histologisch gesichertes gynäkologisches Malignom
- Histologisch gesicherte, nicht-neoplastische Veränderung
- Gesunde altersgematchte Kontrolle
- Zum Zeitpunkt der Diagnose (vor der Operation)
- Vor bzw. während neoadjuvanter und adjuvanter Antiöstrogentherapie, adjuvanter Chemotherapie oder adjuvanter Aromataseinhibitortherapie.

Ausschlusskriterien
- Alter unter 18 Jahren
- Bereits erfolgte Tumorbehandlung

Entsprechende Biochips werden für Diagnose, Voraussage des Therapieverlaufs und Vorhersage der Metastasenbildung entwickelt.

### Beispiel 4:

Für die Entwicklung eines Proteinbiochips für die Diagnose von gynäkologischem Malignom, werden die Ergebnisse der Autoantikörperanalyse mit dem goldenen Standard der Diagnose verglichen und die identifizierten Markersequenzen werden validiert (Markersequenzen für gynäkologisches Malignom). Die Ergebnisse werden dann mit anderen klinischen Charakteristika von gynäkologischem Malignom, beispielsweise Tumorgröße und Malignität korreliert.

### Beispiel 5:

Bei der Entwicklung eines Proteinbiochips zur Voraussage des Therapieverlaufs wird ein bestimmtes Autoantikörperprofil bzw. ein bestimmtes Signal des Proteinbiochips mit dem Ansprechen des gynäkologischen Malignoms auf eine bestimmte Therapie korreliert. Darüber hinaus werden Änderungen des Autoantikörperprofils validiert - auch bezüglich verschiedener Behandlungsoptionen (Continuous time modelling).

### Beispiel 6:

Bei der Entwicklung eines Proteinbiochips für die Prädiktion der Metastasenbildung werden Markersequenzen für gynäkologisches Malignom ausgewählt, die mit Autoantikörpern für gynäkologisches Malignom Wechselwirken, die als Indikator für Metastasenbildung geeignet sind. Durch den Vergleich von Autoantikörpern zum Zeitpunkt der Diagnose von Patientinnen mit und ohne Metastasenbildung können Patientinnen identifiziert werden, die ein hohes Metastaserisiko haben.

Im Rahmen der Identifizierung und Validierung von Markersequenzen für gynäkologisches Malignom können bioinformatische Analysen durchgeführt werden. Für jedes Serum können dazu mittels Microarray Reaktivitäten gegen ca. 2000 unterschiedliche Antigene gemessen werden. Diese Daten werden für ein Ranking der gespotteten Antigene bzgl. ihrer Differenzierungsfähigkeit zwischen gesunden und erkrankten Seren benutzt. Diese Auswertung kann mittels des nicht parametrischen Mann-Whitney Tests auf normalisierten Intensitätsdaten durchgeführt werden. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet.

Desweiteren werden die Daten zur Klassifikation der Seren benutzt. Hierbei kommen unterschiedliche multivariante Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird beispielsweise eine 10fache Cross-Validierung der Daten durchgeführt.

Klonsequenzen (cDNA) SEQ ID No. 1 - 489 siehe Sequenzprotokoll.

**Tabelle A: Daten zu Markersequenzen für gynäkologisches Malignom (RNA) SEQ ID No. 490 - 978.**

| SEQ ID No. | Accession No. | Alias RNA Accession | Blast |
|---|---|---|---|
| 490 | gi\|122114640 | NM_032924.3 | zinc finger protein 3 isoform 2 [Homo sapiens] |
| 491 | gi\|27881505 | NM_007189.1 | ATP-binding cassette sub-family F member 2 isoform a [Homo sapiens] |
| 492 | gi\|194306637 | NM_144985.3 | cadherin-24 isoform 2 [Homo sapiens] |
| 493 | gi\|261337182 | NM_182905.4 | WAS protein family homolog 1 [Homo sapiens] |
| 494 | gi\|16877437 | BC016965.1 | NLRP1 protein [Homo sapiens] |
| 495 | gi\|16332363 | NM_033489.1 | cyclin-dependent kinase 11B isoform 5 [Homo sapiens] |
| 496 | gi\|24797087 | NM_002617.3 | peroxisome biogenesis factor 10 isoform 2 [Homo sapiens] |
| 497 | gi\|51477699 | NM_001003891.1 | mediator of RNA polymerase II transcription subunit 15 isoform a [Homo sapiens] |
| 498 | gi\|215272336 | NM_018667.3 | sphingomyelin phosphodiesterase 3 [Homo sapiens] |
| 499 | gi\|39644878 | BC009967.2 | SCYL1 protein [Homo sapiens] |
| 500 | gi\|156071525 | NM_133171.3 | engulfment and cell motility protein 2 [Homo sapiens] |
| 501 | gi\|145553975 | NM_024657.3 | MORC family CW-type zinc finger protein 4 isoform a [Homo sapiens] |
| 502 | gi\|45387948 | NM_133368.1 | RING finger and SPRY domain-containing protein 1 precursor [Homo sapiens] |
| 503 | gi\|44681483 | NM_152562.2 | cell division cycle-associated protein 2 [Homo sapiens] |
| 504 | gi\|169216540 | XM_001717193.1 | PREDICTED: hypothetical protein [Homo sapiens] |
| 505 | gi\|19584557 | AL713796.1 | hypothetical protein [Homo sapiens] |
| 506 | gi\|55925573 | NM_203404.1 | AMP deaminase 2 isoform 3 [Homo sapiens] |
| 507 | gi\|7020062 | AK000158.1 | unnamed protein product [Homo sapiens] |
| 508 | gi\|41352717 | NM_004416.2 | protein deltex-1 [Homo sapiens] |
| 509 | gi\|190014575 | NM_002775.4 | serine protease HTRA1 precursor [Homo sapiens] |
| 510 | gi\|42544228 | NM_021138.3 | TNF receptor-associated factor 2 [Homo sapiens] |
| 511 | gi\|171543867 | NM_000375.2 | uroporphyrinogen-III synthase [Homo sapiens] |
| 512 | gi\|24307874 | NM_006955.1 | zinc finger protein 33B [Homo sapiens] |
| 513 | gi\|42476323 | NM_003627.4 | large neutral amino acids transporter small subunit 3 [Homo sapiens] |
| 514 | gi\|156071506 | NM_014245.3 | RING-box protein 2 isoform 1 [Homo sapiens] |
| 515 | gi\|44680137 | NM_152414.3 | class E basic helix-loop-helix protein 22 [Homo sapiens] |
| 516 | gi\|154759282 | NM_014603.2 | cerebellar degeneration-related protein 2-like [Homo sapiens] |
| 517 | gi\|156415993 | NM_016037.3 | probable U3 small nucleolar RNA-associated protein 11 [Homo sapiens] |
| 518 | gi\|282847377 | NM_080592.3 | apoptosis-related protein 3 isoform b [Homo sapiens] |
| 519 | gi\|216547614 | NM_019046.2 | ankyrin repeat domain-containing protein 16 isoform a [Homo sapiens] |
| 520 | gi\|54607103 | NM_020187.2 | chromosome 3 open reading frame 37 [Homo sapiens] |
| 521 | gi\|57013271 | NM_001008860.1 | magnesium transporter NIPA2 isoform a [Homo sapiens] |
| 522 | gi\|55770871 | NM_001007102.1 | lethal(3)malignant brain tumor-like protein 3 isoform b [Homo sapiens] |
| 523 | gi\|215599550 | NM_033201.2 | hypothetical protein LOC89927 isoform 1 [Homo sapiens] |
| 524 | gi\|37537683 | NM_007139.2 | zinc finger protein 92 isoform 1 [Homo sapiens] |
| 525 | gi\|116014343 | NM_001624.2 | absent in melanoma 1 protein [Homo sapiens] |
| 526 | gi\|53832030 | NM_004489.4 | G protein pathway suppressor 2 [Homo sapiens] |
| 527 | gi\|18765732 | NM_003081.2 | synaptosomal-associated protein 25 isoform SNAP25A [Homo sapiens] |
| 528 | gi\|195976813 | NM_007117.2 | prothyroliberin precursor [Homo sapiens] |
| 529 | gi\|164519060 | NM_014690.4 | hypothetical protein LOC9715 isoform b [Homo sapiens] |
| 530 | gi\|291327486 | NM_201569.2 | protein SMG7 isoform 4 [Homo sapiens] |
| 531 | gi 40787998 | NM_012446.2 | single-stranded DNA-binding protein 2 [Homo sapiens] |
| 532 | gi\|28416939 | NM_016038.2 | ribosome maturation protein SBDS [Homo sapiens] |
| 533 | gi\|188528682 | NM_021216.4 | endothelial zinc finger protein induced by tumor necrosis factor alpha [Homo sapiens] |
| 534 | gi\|222080063 | NM_032478.3 | 39S ribosomal protein L38, mitochondrial precursor [Homo sapiens] |
| 535 | gi\|186910285 | NM_024570.2 | ribonuclease H2 subunit B isoform 1 [Homo sapiens] |
| 536 | gi\|87298934 | NM_024712.3 | engulfment and cell motility protein 3 [Homo sapiens] |
| 537 | gi\|300068963 | NM_031207.4 | putative hydroxypyruvate isomerase isoform 1 [Homo sapiens] |
| 538 | gi\|226437572 | NM_032360.3 | acyl-CoA-binding domain-containing protein 6 [Homo sapiens] |
| 539 | gi\|119943095 | NM_032364.5 | dnaJ homolog subfamily C member 14 [Homo sapiens] |
| 540 | gi\|333805595 | NM_152652.2 | zinc finger protein 48 isoform 1 [Homo sapiens] |
| 541 | gi\|18375500 | NM_001641.2 | DNA-(apurinic or apyrimidinic site) lyase [Homo sapiens] |
| 542 | gi\|214010180 | NM_001142276.1 | amyloid-like protein 2 isoform 2 [Homo sapiens] |
| 543 | gi\|104487001 | NM_001040712.1 | receptor-type tyrosine-protein phosphatase delta isoform 5 precursor [Homo sapiens] |
| 544 | gi\|32528305 | NM_002913.3 | replication factor C subunit 1 isoform 1 [Homo sapiens] |
| 545 | gi\|41281488 | NM_014761.2 | IST1 homolog [Homo sapiens] |
| 546 | gi\|7023276 | AK001786.1 | unnamed protein product [Homo sapiens] |
| 547 | gi\|217330575 | NM_007225.2 | neurexophilin-3 precursor [Homo sapiens] |
| 548 | gi\|163310748 | NM_001112720.1 | LIM and calponin homology domains-containing protein 1 isoform e [Homo sapiens] |
| 549 | gi\|221219021 | NM_015157.2 | pleckstrin homology-like domain family B member 1 isoform a [Homo sapiens] |
| 550 | gi\|150456423 | NM_015348.1 | transmembrane protein 131 [Homo sapiens] |
| 551 | gi\|62339397 | NM_014604.2 | tax1-binding protein 3 isoform 1 [Homo sapiens] |
| 552 | gi\|66393092 | AY995135.1 | phospholipase C epsilon 1 [Homo sapiens] |
| 553 | gi\|229576807 | NM_020959.2 | anoctamin-8 [Homo sapiens] |
| 554 | gi\|10863994 | NM_021188.1 | zinc finger protein 410 isoform b [Homo sapiens] |
| 555 | gi\|205277444 | NM_021805.2 | single Ig IL-1-related receptor [Homo sapiens] |
| 556 | gi\|205360980 | NM_024557.3 | protein RIC-3 isoform a [Homo sapiens] |
| 557 | gi\|194018548 | NM_030629.2 | C-Maf-inducing protein isoform Tc-mip [Homo sapiens] |
| 558 | gi\|339895851 | NM_001626.4 | RAC-beta serine/threonine-protein kinase isoform 1 [Homo sapiens] |
| 559 | gi\|148613860 | NM_015548.3 | bullous pemphigoid antigen 1 isoform 1eA precursor [Homo sapiens] |
| 560 | gi\|4826685 | NM_004939.1 | ATP-dependent RNA helicase DDX1 [Homo sapiens] |
| 561 | gi\|196115280 | NM_002055.3 | glial fibrillary acidic protein isoform 1 [Homo sapiens] |
| 562 | gi\|156104890 | NM_002096.2 | general transcription factor IIF subunit 1 [Homo sapiens] |
| 563 | gi\|167466172 | NM_005346.4 | heat shock 70 kDa protein 1A/1B [Homo sapiens] |
| 564 | gi\|212276187 | NM_002893.3 | histone-binding protein RBBP7 isoform 2 [Homo sapiens] |
| 565 | gi\|88853067 | NM_002911.3 | regulator of nonsense transcripts 1 [Homo sapiens] |
| 566 | gi\|218083729 | NM_001142684.1 | zinc finger MYM-type protein 5 isoform 3 [Homo sapiens] |
| 567 | gi\|219879804 | NM_006700.2 | TRAF-type zinc finger domain-containing protein 1 [Homo sapiens] |
| 568 | gi\|18379335 | NM_006711.2 | RNA-binding protein with serine-rich domain 1 [Homo sapiens] |
| 569 | gi\|68161542 | NM_007074.2 | coronin-1A [Homo sapiens] |
| 570 | gi\|110227862 | NM_016097.3 | immediate early response 3-interacting protein 1 [Homo sapiens] |
| 571 | gi\|253970487 | NM_018677.3 | acetyl-coenzyme A synthetase, cytoplasmic isoform 1 [Homo sapiens] |
| 572 | gi\|52353964 | NM_020170.3 | nicalin precursor [Homo sapiens] |
| 573 | gi\|116174741 | NM_020223.2 | dentin matrix protein 4 [Homo sapiens] |
| 574 | gi\|187761323 | NM_001127211.1 | shootin-1 isoform a [Homo sapiens] |
| 575 | gi\|38372934 | NM_021948.3 | brevican core protein isoform 1 [Homo sapiens] |
| 576 | gi\|150378519 | NM_032530.1 | zinc finger protein 594 [Homo sapiens] |
| 577 | gi\|81174548 | NM_001037163.1 | STAT3-interacting protein as a repressor [Homo sapiens] |
| 578 | gi\|83921601 | NM_001037984.1 | putative sodium-coupled neutral amino acid transporter 10 isoform a [Homo sapiens] |
| 579 | gi\|222831659 | NM_198475.2 | hypothetical protein LOC284069 precursor [Homo sapiens] |
| 580 | gi\|46519152 | NM_020690.4 | ANKHD1-EIF4EBP3 protein [Homo sapiens] |
| 581 | gi\|166362736 | NM_001114134.1 | erythrocyte membrane protein band 4.2 isoform 2 [Homo sapiens] |
| 582 | gi\|153266933 | NM_002082.3 | G protein-coupled receptor kinase 6 isoform B [Homo sapiens] |
| 583 | gi\|89903006 | NM_002084.3 | glutathione peroxidase 3 precursor [Homo sapiens] |
| 584 | gi\|161353442 | NM_004550.4 | NADH dehydrogenase [ubiquinone] iron-sulfur protein 2, mitochondrial isoform 1 precursor [Homo sapiens] |
| 585 | gi\|23110924 | NM_002798.1 | proteasome subunit beta type-6 precursor [Homo sapiens] |
| 586 | gi\|75750485 | NM_004773.2 | zinc finger HIT domain-containing protein 3 [Homo sapiens] |
| 587 | gi\|215422360 | NM_004786.2 | thioredoxin-like protein 1 [Homo sapiens] |
| 588 | gi\|156071502 | NM_006694.3 | protein JTB precursor [Homo sapiens] |
| 589 | gi\|6996927 | NM_006792.2 | mortality factor 4 [Homo sapiens] |
| 590 | gi\|28373191 | NM_007002.2 | proteasomal ubiquitin receptor ADRM1 precursor [Homo sapiens] |
| 591 | gi\|33188444 | NM_012090.3 | microtubule-actin cross-linking factor 1 isoform a [Homo sapiens] |
| 592 | gi\|34147578 | NM_014338.3 | phosphatidylserine decarboxylase proenzyme [Homo sapiens] |
| 593 | gi\|24308114 | NM_015649.1 | interferon regulatory factor 2-binding protein 1 [Homo sapiens] |
| 594 | gi\|115430234 | NM_001048201.1 | E3 ubiquitin-protein ligase UHRF1 isoform 1 [Homo sapiens] |
| 595 | gi\|56676370 | NM_013291.2 | cleavage and polyadenylation specificity factor subunit 1 [Homo sapiens] |
| 596 | gi\|7706711 | NM_016538.1 | NAD-dependent deacetylase sirtuin-7 [Homo sapiens] |
| 597 | gi\|16554603 | NM_016070.2 | 28S ribosomal protein S23, mitochondrial [Homo sapiens] |
| 598 | gi\|210147465 | NM_022164.2 | tubulointerstitial nephritis antigen-like isoform 1 precursor [Homo sapiens] |
| 599 | gi\|282400941 | NM_022737.2 | lipid phosphate phosphatase-related protein type 2 isoform 1 [Homo sapiens] |
| 600 | gi\|154426254 | NM_032361.2 | THO complex subunit 3 [Homo sapiens] |
| 601 | gi\|34304353 | NM_183244.1 | phosphatase and actin regulator 3 isoform 2 [Homo sapiens] |
| 602 | gi\|194380673 | AK295603.1 | unnamed protein product [Homo sapiens] |
| 603 | gi\|66882523 | NM_001018676.1 | protein farnesyltransferase/geranylgeranyltransferase type-1 subunit alpha isoform b [Homo sapiens] |
| 604 | gi\|38788318 | NM_005275.2 | guanine nucleotide-binding protein-like 1 [Homo sapiens] |
| 605 | gi\|194688131 | NM_002129.3 | high mobility group protein B2 [Homo sapiens] |
| 606 | gi\|156119624 | NM_002215.2 | inter-alpha-trypsin inhibitor heavy chain H1 isoform a [Homo sapiens] |
| 607 | gi\|167614503 | NM_002291.2 | laminin subunit beta-1 precursor [Homo sapiens] |
| 608 | gi\|205277382 | NM_020998.3 | hepatocyte growth factor-like protein precursor [Homo sapiens] |
| 609 | gi\|24430150 | NM_002802.2 | 26S protease regulatory subunit 4 [Homo sapiens] |
| 610 | gi\|5803186 | NM_006755.1 | transaldolase [Homo sapiens] |
| 611 | gi\|31377804 | NM_003425.2 | zinc finger protein 45 [Homo sapiens] |
| 612 | gi\|62739174 | NM_003610.3 | mRNA export factor [Homo sapiens] |
| 613 | gi\|109452590 | NM_003849.2 | succinyl-CoA ligase [GDP-forming] subunit alpha, mitochondrial precursor [Homo sapiens] |
| 614 | gi\|187960106 | NM_003910.3 | protein BUD31 homolog [Homo sapiens] |
| 615 | gi\|117938253 | NM_001077441.1 | bcl-2-associated transcription factor 1 isoform 3 [Homo sapiens] |
| 616 | gi\|86788141 | NM_130442.2 | engulfment and cell motility protein 1 isoform 2 [Homo sapiens] |
| 617 | gi\|260656006 | NM_021102.3 | kunitz-type protease inhibitor 2 isoform a precursor [Homo sapiens] |
| 618 | gi\|4884170 | AL049924.1 | hypothetical protein [Homo sapiens] |
| 619 | gi\|194385125 | AK298842.1 | unnamed protein product [Homo sapiens] |
| 620 | gi\|41352716 | NM_015902.4 | E3 ubiquitin-protein ligase UBR5 [Homo sapiens] |
| 621 | gi\|22035561 | NM_018310.2 | transcription factor IIIB 50 kDa subunit [Homo sapiens] |
| 622 | gi\|122939166 | NM_019613.3 | WD repeat domain phosphoinositide-interacting protein 3 [Homo sapiens] |
| 623 | gi\|151301034 | NM_020151.3 | stAR-related lipid transfer protein 7, mitochondrial precursor [Homo sapiens] |
| 624 | gi\|20127622 | NM_024102.2 | methylosome protein 50 [Homo sapiens] |
| 625 | gi\|75677352 | NM_031921.4 | ATPase family AAA domain-containing protein 3B [Homo sapiens] |
| 626 | gi\|221139702 | NM_031925.2 | transmembrane protein 120A [Homo sapiens] |
| 627 | gi\|238550193 | NM_032298.2 | synaptotagmin-3 [Homo sapiens] |
| 628 | gi\|226437633 | NM_032590.4 | lysine-specific demethylase 2B isoform a [Homo sapiens] |
| 629 | gi\|201025403 | NM_174908.3 | coiled-coil domain-containing protein 50 short isoform [Homo sapiens] |
| 630 | gi\|34335395 | NM_032515.3 | bcl-2-related ovarian killer protein [Homo sapiens] |
| 631 | gi\|28872801 | NM_001212.3 | complement component 1 Q subcomponent-binding protein, mitochondrial precursor [Homo sapiens] |
| 632 | gi\|189339250 | NM_000387.4 | mitochondrial carnitine/acylcarnitine carrier protein [Homo sapiens] |
| 633 | gi\|149999367 | NM_004394.2 | death-associated protein 1 [Homo sapiens] |
| 634 | gi\|55956920 | NM_004499.3 | heterogeneous nuclear ribonucleoprotein A/B isoform b [Homo sapiens] |
| 635 | gi\|62632770 | NM_001015054.1 | DNA-3-methyladenine glycosylase isoform c [Homo sapiens] |
| 636 | gi\|76150622 | NM_006170.2 | putative ribosomal RNA methyltransferase NOP2 [Homo sapiens] |
| 637 | gi\|33239449 | NM_002592.2 | proliferating cell nuclear antigen [Homo sapiens] |
| 638 | gi\|77539056 | NM_003333.3 | ubiquitin-60S ribosomal protein L40 precursor [Homo sapiens] |
| 639 | gi\|190684674 | NM_006297.2 | DNA repair protein XRCC1 [Homo sapiens] |
| 640 | gi\|52630422 | NM_003581.2 | cytoplasmic protein NCK2 isoform A [Homo sapiens] |
| 641 | gi\|197276633 | NM_003707.2 | ruvB-like 1 [Homo sapiens] |
| 642 | gi\|226958573 | NM_004841.3 | ras GTPase-activating protein nGAP isoform 1 [Homo sapiens] |
| 643 | gi\|50428923 | NM_004854.3 | carbohydrate sulfotransferase 10 [Homo sapiens] |
| 644 | gi\|259013555 | NM_004860.3 | fragile X mental retardation syndrome-related protein 2 [Homo sapiens] |
| 645 | gi\|53729351 | NM_005112.4 | WD repeat-containing protein 1 isoform 2 [Homo sapiens] |
| 646 | gi\|6807758 | AL137297.1 | hypothetical protein [Homo sapiens] |
| 647 | gi\|14149701 | NM_015528.1 | E3 ubiquitin-protein ligase RNF167 precursor [Homo sapiens] |
| 648 | gi\|24430138 | NM_015540.2 | RNA polymerase II-associated protein 1 [Homo sapiens] |
| 649 | gi\|187829711 | NM_014417.3 | bcl-2-binding component 3 isoform 4 [Homo sapiens] |
| 650 | gi\|22547135 | NM_015956.2 | 39S ribosomal protein L4, mitochondrial isoform a [Homo sapiens] |
| 651 | gi\|187936926 | NM_014306.4 | tRNA-splicing ligase RtcB homolog [Homo sapiens] |
| 652 | gi\|116063563 | NM_018218.2 | ubiquitin carboxyl-terminal hydrolase 40 [Homo sapiens] |
| 653 | gi\|260166617 | NM_018127.6 | zinc phosphodiesterase ELAC protein 2 isoform 1 [Homo sapiens] |
| 654 | gi\|242247074 | NM_001009877.2 | bromodomain-containing protein 9 isoform 2 [Homo sapiens] |
| 655 | gi\|110349768 | NM_024296.3 | coiled-coil domain-containing protein 28B [Homo sapiens] |
| 656 | gi\|227500152 | NM_024333.2 | fibronectin type III and SPRY domain-containing protein 1 [Homo sapiens] |
| 657 | gi\|33337995 | AF173977.1 | MSTP107 [Homo sapiens] |
| 658 | gi\|31377666 | NM_031490.2 | Ion protease homolog 2, peroxisomal [Homo sapiens] |
| 659 | gi\|206597512 | NM_015242.4 | arf-GAP with Rho-GAP domain, ANK repeat and PH domain-containing protein 1 isoform a [Homo sapiens] |
| 660 | gi\|45387954 | NM_205767.1 | protein QIL1 [Homo sapiens] |
| 661 | gi\|148596939 | NM_152383.4 | DIS3-like exonuclease 2 [Homo sapiens] |
| 662 | gi\|42794621 | NM_203374.1 | zinc finger protein 784 [Homo sapiens] |
| 663 | gi\|113424118 | XM_370711.5 | PREDICTED: similar to FRAS1 related extracellular matrix protein 2 [Homo sapiens] |
| 664 | gi\|45446741 | NM_001354.4 | aldo-keto reductase family 1 member C2 isoform 1 [Homo sapiens] |
| 665 | gi\|100913205 | NM_001357.3 | ATP-dependent RNA helicase A [Homo sapiens] |
| 666 | gi\|215276985 | NM_145740.3 | glutathione S-transferase A1 [Homo sapiens] |
| 667 | gi\|31657145 | NM_033453.2 | inosine triphosphate pyrophosphatase isoform a [Homo sapiens] |
| 668 | gi\|89276761 | NM_032940.2 | DNA-directed RNA polymerase II subunit RPB3 [Homo sapiens] |
| 669 | gi\|110618252 | NM_005035.3 | DNA-directed RNA polymerase, mitochondrial precursor [Homo sapiens] |
| 670 | gi\|42794609 | NM_002939.3 | ribonuclease inhibitor [Homo sapiens] |
| 671 | gi\|157266329 | NM_001033.3 | ribonucleoside-diphosphate reductase large subunit [Homo sapiens] |
| 672 | gi\|71051615 | NM_000374.3 | uroporphyrinogen decarboxylase [Homo sapiens] |
| 673 | gi\|45269143 | NM_012289.3 | kelch-like ECH-associated protein 1 [Homo sapiens] |
| 674 | gi\|194294518 | NM_001130102.1 | oxysterols receptor LXR-alpha isoform c [Homo sapiens] |
| 675 | gi\|7706336 | NM_016057.1 | coatomer subunit zeta-1 [Homo sapiens] |
| 676 | gi\|122114657 | NM_015037.2 | hypothetical protein LOC23053 isoform 1 [Homo sapiens] |
| 677 | gi\|150456431 | NM_001099436.1 | serine/threonine-protein kinase ULK3 [Homo sapiens] |
| 678 | gi\|118572609 | NM_016183.3 | mRNA turnover protein 4 homolog [Homo sapiens] |
| 679 | gi\|116812574 | NM_016258.2 | YTH domain family protein 2 isoform 1 [Homo sapiens] |
| 680 | gi\|21327682 | NM_032630.2 | cyclin-dependent kinase 2-interacting protein isoform 2 [Homo sapiens] |
| 681 | gi\|62526025 | NM_001014840.1 | protein CutA isoform 3 [Homo sapiens] |
| 682 | gi\|68533248 | NM_018476.3 | protein BEX1 [Homo sapiens] |
| 683 | gi\|94818878 | NM_001040457.1 | rhomboid domain-containing protein 2 isoform b [Homo sapiens] |
| 684 | gi\|83779009 | NM_024077.3 | selenocysteine insertion sequence-binding protein 2 [Homo sapiens] |
| 685 | gi\|208431768 | NM_031450.3 | basophilic leukemia expressed protein BLES03 isoform 2 [Homo sapiens] |
| 686 | gi\|32306518 | NM_031492.2 | RNA-binding protein 4B [Homo sapiens] |
| 687 | gi\|40317613 | NM_138777.2 | ribosome-recycling factor, mitochondrial isoform 1 precursor [Homo sapiens] |
| 688 | gi\|194473692 | NM_138340.4 | abhydrolase domain-containing protein 3 [Homo sapiens] |
| 689 | gi\|34304361 | NM_015102.2 | nephrocystin-4 [Homo sapiens] |
| 690 | gi\|49169840 | NM_001001795.1 | hypothetical protein LOC414919 [Homo sapiens] |
| 691 | gi\|89276783 | NM_001617.2 | beta-adducin isoform a [Homo sapiens] |
| 692 | gi\|148539875 | NM_001619.3 | beta-adrenergic receptor kinase 1 [Homo sapiens] |
| 693 | gi\|33591068 | NM_000386.2 | bleomycin hydrolase [Homo sapiens] |
| 694 | gi\|55770843 | NM_001903.2 | catenin alpha-1 [Homo sapiens] |
| 695 | gi\|40288196 | NM_005257.3 | transcription factor GATA-6 [Homo sapiens] |
| 696 | gi\|85838503 | NM_005318.3 | histone H1.0 [Homo sapiens] |
| 697 | gi\|194294499 | NM_005341.2 | zinc finger and BTB domain-containing protein 48 [Homo sapiens] |
| 698 | gi\|170014687 | NM_000414.2 | peroxisomal multifunctional enzyme type 2 isoform 2 [Homo sapiens] |
| 699 | gi\|33356546 | NM_004526.2 | DNA replication licensing factor MCM2 [Homo sapiens] |
| 700 | gi\|29826281 | NM_177983.1 | protein phosphatase 1G [Homo sapiens] |
| 701 | gi\|68216257 | NM_000981.3 | 60S ribosomal protein L19 [Homo sapiens] |
| 702 | gi\|197209833 | NM_005066.2 | splicing factor, proline- and glutamine-rich [Homo sapiens] |
| 703 | gi\|86991437 | NM_001039465.1 | serine/arginine-rich splicing factor 5 [Homo sapiens] |
| 704 | gi\|170014702 | NM_003023.4 | SH3 domain-binding protein 2 isoform a [Homo sapiens] |
| 705 | gi\|40806158 | NM_003250.4 | thyroid hormone receptor alpha isoform 2 [Homo sapiens] |
| 706 | gi\|31543803 | NM_006528.2 | tissue factor pathway inhibitor 2 precursor [Homo sapiens] |
| 707 | gi\|219842276 | NM_005439.2 | myeloid leukemia factor 2 [Homo sapiens] |
| 708 | gi\|201860299 | NM_003977.2 | AH receptor-interacting protein [Homo sapiens] |
| 709 | gi\|209862763 | NM_014806.2 | iporin [Homo sapiens] |
| 710 | gi\|124378038 | NM_014866.1 | protein transport protein Secl6A [Homo sapiens] |
| 711 | gi\|31652256 | NM_005461.3 | transcription factor MafB [Homo sapiens] |
| 712 | gi\|32307182 | NM_006379.2 | semaphorin-3C precursor [Homo sapiens] |
| 713 | gi\|32454736 | NM_033278.2 | tripartite motif-containing protein 3 [Homo sapiens] |
| 714 | gi\|260656035 | NM_006702.4 | neuropathy target esterase isoform b [Homo sapiens] |
| 715 | gi\|21758255 | AK098275.1 | unnamed protein product [Homo sapiens] |
| 716 | gi\|197313761 | NM_012137.3 | N(G),N(G)-dimethylarginine dimethylaminohydrolase 1 isoform 1 [Homo sapiens] |
| 717 | gi\|223468619 | NM_013446.3 | E3 ubiquitin-protein ligase makorin-1 isoform 1 [Homo sapiens] |
| 718 | gi\|11385647 | AF273045.1 | CTCL tumor antigen se14-3 [Homo sapiens] |
| 719 | gi\|196115157 | NM_001131018.1 | cip1-interacting zinc finger protein isoform 4 [Homo sapiens] |
| 720 | gi\|55953121 | NM_015705.4 | small G protein signaling modulator 3 [Homo sapiens] |
| 721 | gi\|7020524 | AK000437.1 | unnamed protein product [Homo sapiens] |
| 722 | gi\|217035104 | NM_018198.3 | dnaJ homolog subfamily C member 11 [Homo sapiens] |
| 723 | gi\|34147349 | NM_024042.2 | meteorin precursor [Homo sapiens] |
| 724 | gi\|117320542 | NM_001077497.1 | proline-rich protein 3 isoform b [Homo sapiens] |
| 725 | gi\|113204623 | NM_032193.3 | ribonuclease H2 subunit C [Homo sapiens] |
| 726 | gi\|20270302 | NM_138769.1 | mitochondrial Rho GTPase 2 [Homo sapiens] |
| 727 | gi\|16445355 | NM_033415.2 | armadillo repeat-containing protein 6 isoform 2 [Homo sapiens] |
| 728 | gi\|62244043 | NM_001014446.1 | OCIA domain-containing protein 2 isoform 1 [Homo sapiens] |
| 729 | gi\|333609247 | NM_153269.2 | hypothetical protein LOC140680 isoform 1 [Homo sapiens] |
| 730 | gi\|21389514 | NM_144726.1 | RING finger protein 145 isoform 2 [Homo sapiens] |
| 731 | gi\|197276595 | NM_006129.3 | bone morphogenetic protein 1 isoform 3 precursor [Homo sapiens] |
| 732 | gi\|169790898 | NM_001122630.1 | cyclin-dependent kinase inhibitor 1C isoform b [Homo sapiens] |
| 733 | gi\|66346646 | NM_001908.3 | cathepsin B preproprotein [Homo sapiens] |
| 734 | gi\|38201713 | NM_001419.2 | ELAV-like protein 1 [Homo sapiens] |
| 735 | gi\|46411186 | NM_005234.3 | nuclear receptor subfamily 2 group F member 6 [Homo sapiens] |
| 736 | gi\|167000329 | NM_021903.2 | gamma-aminobutyric acid type B receptor subunit 1 isoform b precursor [Homo sapiens] |
| 737 | gi\|27502382 | NM_172316.1 | homeobox protein Meis2 isoform h [Homo sapiens] |
| 738 | gi\|51317374 | NM_005008.2 | NHP2-like protein 1 [Homo sapiens] |
| 739 | gi\|20072756 | BC027470.1 | PCDHGC3 protein [Homo sapiens] |
| 740 | gi\|25777611 | NM_002809.2 | 26S proteasome non-ATPase regulatory subunit 3 [Homo sapiens] |
| 741 | gi\|125987582 | NM_080840.2 | receptor-type tyrosine-protein phosphatase alpha isoform 2 precursor [Homo sapiens] |
| 742 | gi\|21536317 | NM_006268.3 | zinc finger protein ubi-d4 [Homo sapiens] |
| 743 | gi\|194306565 | NM_007370.4 | replication factor C subunit 5 isoform 1 [Homo sapiens] |
| 744 | gi\|55925647 | NM_000994.3 | 60S ribosomal protein L32 [Homo sapiens] |
| 745 | gi\|42476326 | NM_003025.2 | endophilin-A2 isoform 1 [Homo sapiens] |
| 746 | gi\|56699493 | NM_003179.2 | synaptophysin [Homo sapiens] |
| 747 | gi\|83281440 | NM_003755.3 | eukaryotic translation initiation factor 3 subunit G [Homo sapiens] |
| 748 | gi\|331284175 | NM_001077261.3 | nuclear receptor corepressor 2 isoform 2 [Homo sapiens] |
| 749 | gi\|33519473 | NM_005831.3 | calcium-binding and coiled-coil domain-containing protein 2 [Homo sapiens] |
| 750 | gi\|40068460 | NM_007284.3 | twinfilin-2 [Homo sapiens] |
| 751 | gi\|19913459 | BC026067.1 | WDSOF1 protein [Homo sapiens] |
| 752 | gi\|155030235 | NM_001100412.1 | ribonuclease 3 isoform 2 [Homo sapiens] |
| 753 | gi\|46370087 | NM_013306.3 | sorting nexin-15 isoform A [Homo sapiens] |
| 754 | gi\|109134346 | NM_001042399.1 | coiled-coil domain-containing protein 41 [Homo sapiens] |
| 755 | gi\|50409706 | NM_016638.2 | ADP-ribosylation factor-like protein 6-interacting protein 4 isoform 2 [Homo sapiens] |
| 756 | gi\|19913437 | NM_015994.2 | V-type proton ATPase subunit D [Homo sapiens] |
| 757 | gi\|85362736 | NM_001039140.1 | hypothetical protein LOC54976 [Homo sapiens] |
| 758 | gi\|21750404 | AK091925.1 | unnamed protein product [Homo sapiens] |
| 759 | gi\|157388940 | NM_017998.2 | hypothetical protein LOC55071 [Homo sapiens] |
| 760 | gi\|189095252 | NM_001080425.2 | protein BEX4 [Homo sapiens] |
| 761 | gi\|46559760 | NM_032512.2 | PDZ domain-containing protein 4 [Homo sapiens] |
| 762 | gi\|198041727 | NM_001134774.1 | kinesin light chain 2 isoform 2 [Homo sapiens] |
| 763 | gi\|55749441 | NM_001006935.1 | transcription elongation factor A protein-like 4 [Homo sapiens] |
| 764 | gi\|18027791 | AF318350.1 | unknown [Homo sapiens] |
| 765 | gi\|116235475 | NM_032856.2 | WD repeat-containing protein 73 [Homo sapiens] |
| 766 | gi\|91176336 | NM_138383.2 | MTSS1-like protein [Homo sapiens] |
| 767 | gi\|156523245 | NM_145239.2 | proline-rich transmembrane protein 2 [Homo sapiens] |
| 768 | gi\|209571529 | NM_182527.2 | calcium-binding protein 7 [Homo sapiens] |
| 769 | gi\|195947381 | NM_178314.3 | RILP-like protein 1 [Homo sapiens] |
| 770 | gi\|34147601 | NM_004309.3 | rho GDP-dissociation inhibitor 1 isoform a [Homo sapiens] |
| 771 | gi\|23110949 | NM_001909.3 | cathepsin D preproprotein [Homo sapiens] |
| 772 | gi\|168480109 | NM_005225.2 | transcription factor E2F1 [Homo sapiens] |
| 773 | gi\|188497702 | NM_004186.3 | semaphorin-3F precursor [Homo sapiens] |
| 774 | gi\|109150415 | NM_012293.1 | peroxidasin homolog precursor [Homo sapiens] |
| 775 | gi\|115430228 | NM_005132.2 | meiotic recombination protein REC8 homolog [Homo sapiens] |
| 776 | gi\|24497575 | NM_006066.2 | alcohol dehydrogenase [NADP+] [Homo sapiens] |
| 777 | gi\|5453837 | NM_006396.1 | Sjoegren syndrome/scleroderma autoantigen 1 [Homo sapiens] |
| 778 | gi\|6005793 | NM_007213.1 | PRA1 family protein 2 [Homo sapiens] |
| 779 | gi\|209870093 | NM_012437.4 | SNARE-associated protein Snapin [Homo sapiens] |
| 780 | gi\|148664189 | NM_014333.3 | cell adhesion molecule 1 isoform 1 [Homo sapiens] |
| 781 | gi\|18496982 | NM_015526.1 | CAP-Gly domain-containing linker protein 3 [Homo sapiens] |
| 782 | gi\|238624145 | NM_017455.3 | neuroplastin isoform a precursor [Homo sapiens] |
| 783 | gi\|148833501 | NM_016403.3 | protein CWC15 homolog [Homo sapiens] |
| 784 | gi\|10438635 | AK025958.1 | unnamed protein product [Homo sapiens] |
| 785 | gi\|58761526 | NM_018181.4 | zinc finger protein 532 [Homo sapiens] |
| 786 | gi\|8922734 | NM_018255.1 | elongator complex protein 2 isoform 2 [Homo sapiens] |
| 787 | gi\|41327716 | NM_022104.3 | phosphorylated CTD-interacting factor 1 [Homo sapiens] |
| 788 | gi\|40068484 | NM_022834.3 | von Willebrand factor A domain-containing protein 1 isoform 1 precursor [Homo sapiens] |
| 789 | gi\|215820629 | NM_032355.3 | vacuolar fusion protein MON1 homolog A isoform a [Homo sapiens] |
| 790 | gi\|126090574 | NM_001039503.2 | serine protease 53 precursor [Homo sapiens] |
| 791 | gi\|24307878 | NM_001378.1 | cytoplasmic dynein 1 intermediate chain 2 [Homo sapiens] |
| 792 | gi\|153791534 | NM_021078.2 | histone acetyltransferase KAT2A [Homo sapiens] |
| 793 | gi\|73747843 | NM_002311.3 | DNA ligase 3 isoform beta precursor [Homo sapiens] |
| 794 | gi\|38045911 | NM_000269.2 | nucleoside diphosphate kinase A isoform b [Homo sapiens] |
| 795 | gi\|219879812 | NM_002696.2 | DNA-directed RNA polymerase II subunit RPB7 [Homo sapiens] |
| 796 | gi\|56790298 | NM_002779.3 | PH and SEC7 domain-containing protein 1 [Homo sapiens] |
| 797 | gi\|21359853 | NM_004582.2 | geranylgeranyl transferase type-2 subunit beta [Homo sapiens] |
| 798 | gi\|194394229 | NM_003313.3 | GDP-L-fucose synthase [Homo sapiens] |
| 799 | gi\|221136767 | NM_000371.3 | transthyretin precursor [Homo sapiens] |
| 800 | gi\|193211615 | NM_003634.2 | protein NipSnap homolog 1 isoform 1 [Homo sapiens] |
| 801 | gi\|21396488 | NM_004793.2 | Ion protease homolog, mitochondrial precursor [Homo sapiens] |
| 802 | gi\|24234719 | NM_005494.2 | dnaJ homolog subfamily B member 6 isoform b [Homo sapiens] |
| 803 | gi\|86792514 | NM_005700.3 | dipeptidyl peptidase 3 [Homo sapiens] |
| 804 | gi\|34335235 | NM_006651.3 | complexin-1 [Homo sapiens] |
| 805 | gi\|116805324 | NM_170713.2 | ras association domain-containing protein 1 isoform C [Homo sapiens] |
| 806 | gi\|114796625 | NM_013356.2 | monocarboxylate transporter 3 [Homo sapiens] |
| 807 | gi\|95089415 | NM_012478.3 | WW domain-binding protein 2 [Homo sapiens] |
| 808 | gi\|51094102 | NM_019082.2 | probable ATP-dependent RNA helicase DDX56 [Homo sapiens] |
| 809 | gi\|33859677 | NM_017879.1 | zinc finger protein 416 [Homo sapiens] |
| 810 | gi\|24432025 | NM_032775.2 | kelch-like protein 22 [Homo sapiens] |
| 811 | gi\|126273571 | NM_144586.5 | ly6/PLAUR domain-containing protein 1 isoform a [Homo sapiens] |
| 812 | gi\|149363677 | NM_199287.2 | coiled-coil domain-containing protein 137 [Homo sapiens] |
| 813 | gi\|148763344 | NM_033529.2 | cyclin-dependent kinase 11A isoform 4 [Homo sapiens] |
| 814 | gi\|38372924 | NM_198589.1 | basigin isoform 2 precursor [Homo sapiens] |
| 815 | gi\|40804467 | NM_000723.3 | voltage-dependent L-type calcium channel subunit beta-1 isoform 1 [Homo sapiens] |
| 816 | gi\|40549399 | NM_001894.4 | casein kinase I isoform epsilon [Homo sapiens] |
| 817 | gi\|34486089 | NM_004152.2 | ornithine decarboxylase antizyme 1 [Homo sapiens] |
| 818 | gi\|189458830 | NM_002880.3 | RAF proto-oncogene serine/threonine-protein kinase [Homo sapiens] |
| 819 | gi\|71164880 | NM_001011.3 | 40S ribosomal protein S7 [Homo sapiens] |
| 820 | gi\|71164876 | NM_001014.3 | 40S ribosomal protein S10 [Homo sapiens] |
| 821 | gi\|48255969 | NM_002969.3 | mitogen-activated protein kinase 12 [Homo sapiens] |
| 822 | gi\|56117849 | NM_007158.4 | cold shock domain-containing protein E1 isoform 2 [Homo sapiens] |
| 823 | gi\|149158693 | NM_080702.2 | large proline-rich protein BAG6 isoform b [Homo sapiens] |
| 824 | gi\|75992939 | NM_004651.3 | ubiquitin carboxyl-terminal hydrolase 11 [Homo sapiens] |
| 825 | gi\|156071485 | NM_003680.3 | tyrosyl-tRNA synthetase, cytoplasmic [Homo sapiens] |
| 826 | gi\|195972858 | NM_004228.5 | cytohesin-2 isoform 2 [Homo sapiens] |
| 827 | gi\|49472826 | NM_006324.2 | craniofacial development protein 1 [Homo sapiens] |
| 828 | gi\|61744459 | NM_017588.2 | WD repeat-containing protein 5 [Homo sapiens] |
| 829 | gi\|31543548 | NM_007273.3 | prohibitin-2 isoform 2 [Homo sapiens] |
| 830 | gi\|27374999 | NM_007285.6 | gamma-aminobutyric acid receptor-associated protein-like 2 [Homo sapiens] |
| 831 | gi\|38146101 | NM_013274.2 | DNA polymerase lambda isoform a [Homo sapiens] |
| 832 | gi\|171906572 | NM_014063.6 | drebrin-like protein isoform a [Homo sapiens] |
| 833 | gi\|17978480 | NM_080413.1 | vacuolar protein sorting-associated protein 16 homolog isoform 3 [Homo sapiens] |
| 834 | gi\|34147350 | NM_023940.2 | ras-like protein family member 11B [Homo sapiens] |
| 835 | gi\|190194415 | NM_024326.3 | F-box/LRR-repeat protein 15 [Homo sapiens] |
| 836 | gi\|16604251 | NM_052860.1 | zinc finger protein 300 isoform 2 [Homo sapiens] |
| 837 | gi\|115511029 | NM_138441.2 | protein MB21D1 [Homo sapiens] |
| 838 | gi\|23510412 | NM_052948.2 | rho GTPase-activating protein 33 isoform 1 [Homo sapiens] |
| 839 | gi\|223890145 | NM_182498.3 | zinc finger protein 428 [Homo sapiens] |
| 840 | gi\|58761495 | NM_001011724.1 | heterogeneous nuclear ribonucleoprotein A1-like 2 [Homo sapiens] |
| 841 | gi\|34530768 | AK124865.1 | unnamed protein product [Homo sapiens] |
| 842 | gi\|197100212 | NM_001610.2 | lysosomal acid phosphatase isoform 1 precursor [Homo sapiens] |
| 843 | gi\|34452697 | NM_004924.3 | alpha-actinin-4 [Homo sapiens] |
| 844 | gi\|17017985 | NM_001861.2 | cytochrome c oxidase subunit 4 isoform 1, mitochondrial precursor [Homo sapiens] |
| 845 | gi\|197245333 | NM_005273.3 | guanine nucleotide-binding protein G(I)/G(S)/G(T) subunit beta-2 [Homo sapiens] |
| 846 | gi\|21071025 | NM_005319.3 | histone H1.2 [Homo sapiens] |
| 847 | gi\|188497749 | NM_033500.2 | hexokinase-1 isoform HKI-td [Homo sapiens] |
| 848 | gi\|250083 | S37374.1 | HSJ1b [Homo sapiens] |
| 849 | gi\|219842228 | NM_004537.4 | nucleosome assembly protein 1-like 1 [Homo sapiens] |
| 850 | gi\|45439320 | NM_005038.2 | peptidyl-prolyl cis-trans isomerase D [Homo sapiens] |
| 851 | gi\|207029438 | NM_001135256.1 | histone-binding protein RBBP4 isoform c [Homo sapiens] |
| 852 | gi\|171543894 | NM_002973.3 | ataxin-2 [Homo sapiens] |
| 853 | gi\|51477701 | NM_001003801.1 | SWI/SNF-related matrix-associated actin-dependent regulator of chromatin subfamily D member 3 isoform 2 [Homo sapiens] |
| 854 | gi\|54607088 | NM_001005914.1 | semaphorin-3B isoform 2 precursor [Homo sapiens] |
| 855 | gi\|215820638 | NM_014714.3 | intraflagellar transport protein 140 homolog [Homo sapiens] |
| 856 | gi\|45643136 | NM_006791.2 | mortality factor 4-like protein 1 isoform 1 [Homo sapiens] |
| 857 | gi\|115511037 | NM_001009955.2 | single-stranded DNA-binding protein 3 isoform c [Homo sapiens] |
| 858 | gi\|194248096 | NM_013333.3 | epsin-1 isoform c [Homo sapiens] |
| 859 | gi\|223941871 | NM_017825.2 | poly(ADP-ribose) glycohydrolase ARH3 [Homo sapiens] |
| 860 | gi\|268370292 | NM_020442.4 | valyl-tRNA synthetase, mitochondrial isoform 2 precursor [Homo sapiens] |
| 861 | gi\|53831987 | NM_020659.2 | protein tweety homolog 1 isoform 1 [Homo sapiens] |
| 862 | gi\|165932369 | NM_024582.4 | protocadherin Fat 4 precursor [Homo sapiens] |
| 863 | gi\|52145308 | NM_032808.5 | leucine-rich repeat neuronal 6A [Homo sapiens] |
| 864 | gi\|150417982 | NM_001606.4 | ATP-binding cassette sub-family A member 2 isoform a [Homo sapiens] |
| 865 | gi\|71565153 | NM_000671.3 | alcohol dehydrogenase class-3 [Homo sapiens] |
| 866 | gi\|156523967 | NM_001618.3 | poly [ADP-ribose] polymerase 1 [Homo sapiens] |
| 867 | gi\|49249971 | NM_152296.3 | sodium/potassium-transporting ATPase subunit alpha-3 [Homo sapiens] |
| 868 | gi\|82546842 | NM_004327.3 | breakpoint cluster region protein isoform 1 [Homo sapiens] |
| 869 | gi\|20149593 | NM_007355.2 | heat shock protein HSP 90-beta [Homo sapiens] |
| 870 | gi\|27436949 | NM_005573.2 | lamin-B1 isoform 1 [Homo sapiens] |
| 871 | gi\|61742795 | NM_005581.3 | basal cell adhesion molecule isoform 1 precursor [Homo sapiens] |
| 872 | gi\|121256637 | NM_000918.3 | protein disulfide-isomerase precursor [Homo sapiens] |
| 873 | gi\|209529732 | NM_139032.2 | mitogen-activated protein kinase 7 isoform 2 [Homo sapiens] |
| 874 | gi\|257196241 | NM_001063.3 | serotransferrin precursor [Homo sapiens] |
| 875 | gi\|124028528 | NM_004819.2 | symplekin [Homo sapiens] |
| 876 | gi\|253735771 | NM_004723.3 | rho guanine nucleotide exchange factor 2 isoform 3 [Homo sapiens] |
| 877 | gi\|115298667 | NM_004814.2 | U5 small nuclear ribonucleoprotein 40 kDa protein [Homo sapiens] |
| 878 | gi\|148922919 | NM_014856.2 | DENN domain-containing protein 4B [Homo sapiens] |
| 879 | gi\|222080097 | NM_017450.2 | brain-specific angiogenesis inhibitor 1-associated protein 2 isoform 1 [Homo sapiens] |
| 880 | gi\|38455426 | NM_006430.2 | T-complex protein 1 subunit delta [Homo sapiens] |
| 881 | gi\|300192932 | NM_006796.2 | AFG3-like protein 2 [Homo sapiens] |
| 882 | gi\|166795249 | NM_006845.3 | kinesin-like protein KIF2C [Homo sapiens] |
| 883 | gi\|21396479 | NM_007367.2 | RNA-binding protein Raly short isoform [Homo sapiens] |
| 884 | gi\|150010638 | NM_015276.1 | ubiquitin carboxyl-terminal hydrolase 22 [Homo sapiens] |
| 885 | gi\|82659108 | NM_020765.2 | E3 ubiquitin-protein ligase UBR4 [Homo sapiens] |
| 886 | gi\|151101234 | NM_012461.2 | TERF1-interacting nuclear factor 2 isoform 2 [Homo sapiens] |
| 887 | gi\|134284354 | NM_012336.3 | nuclear prelamin A recognition factor isoform a [Homo sapiens] |
| 888 | gi\|50726984 | NM_016337.2 | ena/VASP-like protein [Homo sapiens] |
| 889 | gi\|37622893 | NM_194460.1 | RING finger protein 126 [Homo sapiens] |
| 890 | gi\|20521787 | AB033013.2 | KIAA1187 protein [Homo sapiens] |
| 891 | gi\|112789552 | NM_020820.3 | phosphatidylinositol 3,4,5-trisphosphate-dependent Rac exchanger 1 protein [Homo sapiens] |
| 892 | gi\|224549001 | NM_032905.4 | splicing factor 45 [Homo sapiens] |
| 893 | gi\|209862908 | NM_001136053.1 | transmembrane protein adipocyte-associated 1 isoform 1 [Homo sapiens] |
| 894 | gi\|197102773 | NM_007099.3 | low molecular weight phosphotyrosine protein phosphatase isoform b [Homo sapiens] |
| 895 | gi\|239937553 | NM_000687.2 | adenosylhomocysteinase isoform 1 [Homo sapiens] |
| 896 | gi\|18201904 | NM_000175.2 | glucose-6-phosphate isomerase isoform 2 [Homo sapiens] |
| 897 | gi\|169259765 | NM_001514.5 | transcription initiation factor IIB [Homo sapiens] |
| 898 | gi\|187761305 | NM_002134.3 | heme oxygenase 2 [Homo sapiens] |
| 899 | gi\|10434001 | AK022548.1 | unnamed protein product [Homo sapiens] |
| 900 | gi\|24797084 | NM_002265.4 | importin subunit beta-1 [Homo sapiens] |
| 901 | gi\|156631004 | NM_002812.4 | 26S proteasome non-ATPase regulatory subunit 8 [Homo sapiens] |
| 902 | gi\|154759258 | NM_003127.2 | spectrin alpha chain, brain isoform 2 [Homo sapiens] |
| 903 | gi\|38505154 | NM_003195.4 | transcription elongation factor A protein 2 isoform a [Homo sapiens] |
| 904 | gi\|141801911 | NM_003295.2 | translationally-controlled tumor protein [Homo sapiens] |
| 905 | gi\|21396499 | NM_003609.2 | HIRA-interacting protein 3 [Homo sapiens] |
| 906 | gi\|83367070 | NM_003753.3 | eukaryotic translation initiation factor 3 subunit D [Homo sapiens] |
| 907 | gi\|83367079 | NM_003801.3 | glycosylphosphatidylinositol anchor attachment 1 protein [Homo sapiens] |
| 908 | gi\|33469915 | NM_003906.3 | 80 kDa MCM3-associated protein [Homo sapiens] |
| 909 | gi\|52486264 | NM_006029.4 | paraneoplastic antigen Ma1 [Homo sapiens] |
| 910 | gi\|34365370 | BX641004.1 | hypothetical protein [Homo sapiens] |
| 911 | gi\|215490016 | NM_012286.2 | mortality factor 4-like protein 2 [Homo sapiens] |
| 912 | gi\|148727367 | NM_015695.2 | bromodomain and PHD finger-containing protein 3 [Homo sapiens] |
| 913 | gi\|193788635 | NM_032687.3 | cysteine and histidine-rich protein 1 isoform 2 [Homo sapiens] |
| 914 | gi\|50345274 | NM_016185.2 | hematological and neurological expressed 1 protein isoform 1 [Homo sapiens] |
| 915 | gi\|56788357 | NM_020243.4 | mitochondrial import receptor subunit TOM22 homolog [Homo sapiens] |
| 916 | gi\|32455272 | NM_006648.3 | serine/threonine-protein kinase WNK2 [Homo sapiens] |
| 917 | gi\|119964727 | NM_152783.3 | D-2-hydroxyglutarate dehydrogenase, mitochondrial precursor [Homo sapiens] |
| 918 | gi\|114520614 | NM_001954.4 | epithelial discoidin domain-containing receptor 1 isoform 1 precursor [Homo sapiens] |
| 919 | gi\|62241006 | NM_001888.2 | mu-crystallin homolog isoform 1 [Homo sapiens] |
| 920 | gi\|206725516 | NM_000801.3 | peptidyl-prolyl cis-trans isomerase FKBP1A isoform a [Homo sapiens] |
| 921 | gi\|14505488 | NM_002539.1 | ornithine decarboxylase [Homo sapiens] |
| 922 | gi\|38679891 | NM_006223.2 | peptidyl-prolyl cis-trans isomerase NIMA-interacting 4 isoform 1 [Homo sapiens] |
| 923 | gi\|194018536 | NM_002766.2 | phosphoribosyl pyrophosphate synthase-associated protein 1 [Homo sapiens] |
| 924 | gi\|78217389 | NM_001004.3 | 60S acidic ribosomal protein P2 [Homo sapiens] |
| 925 | gi\|197927095 | NM_001030.4 | 40S ribosomal protein S27 [Homo sapiens] |
| 926 | gi\|54792063 | NM_003352.4 | small ubiquitin-related modifier 1 isoform a precursor [Homo sapiens] |
| 927 | gi\|31083149 | NM_003502.2 | axin-1 isoform a [Homo sapiens] |
| 928 | gi\|153792767 | NM_021195.4 | claudin-6 [Homo sapiens] |
| 929 | gi\|151108508 | NM_014859.4 | rho GTPase-activating protein 44 [Homo sapiens] |
| 930 | gi\|22907051 | NM_006409.2 | actin-related protein 2/3 complex subunit 1A isoform 1 [Homo sapiens] |
| 931 | gi\|54112115 | NM_006802.2 | splicing factor 3A subunit 3 [Homo sapiens] |
| 932 | gi\|209413761 | NM_017586.2 | calcium channel flower homolog isoform a [Homo sapiens] |
| 933 | gi\|209954817 | NM_014921.4 | latrophilin-1 isoform 2 precursor [Homo sapiens] |
| 934 | gi\|14591905 | NM_012423.2 | 60S ribosomal protein L13a [Homo sapiens] |
| 935 | gi\|38570153 | NM_012279.2 | zinc finger protein 346 [Homo sapiens] |
| 936 | gi\|44680150 | NM_014335.2 | EP300-interacting inhibitor of differentiation 1 [Homo sapiens] |
| 937 | gi\|166197689 | NM_001114090.1 | ephexin-1 isoform 2 [Homo sapiens] |
| 938 | gi\|75677342 | NM_015544.2 | transmembrane protein 98 [Homo sapiens] |
| 939 | gi\|27414496 | NM_014153.2 | zinc finger CCCH domain-containing protein 7A [Homo sapiens] |
| 940 | gi\|8922866 | NM_018322.1 | hypothetical protein LOC55776 [Homo sapiens] |
| 941 | gi\|142383690 | NM_022756.4 | chromatin modification-related protein MEAF6 [Homo sapiens] |
| 942 | gi\|186928845 | NM_032476.3 | 28S ribosomal protein S6, mitochondrial [Homo sapiens] |
| 943 | gi\|141872402 | NM_201398.1 | FAD synthase isoform 2 [Homo sapiens] |
| 944 | gi\|197304706 | NM_032350.5 | hypothetical protein LOC84310 [Homo sapiens] |
| 945 | gi\|56786146 | NM_001801.2 | cysteine dioxygenase type 1 [Homo sapiens] |
| 946 | gi\|55750040 | NM_001940.3 | atrophin-1 [Homo sapiens] |
| 947 | gi\|194097322 | NM_004092.3 | enoyl-CoA hydratase, mitochondrial precursor [Homo sapiens] |
| 948 | gi\|160420313 | NM_001456.3 | filamin-A isoform 1 [Homo sapiens] |
| 949 | gi\|89276752 | NM_198252.2 | gelsolin isoform b [Homo sapiens] |
| 950 | gi\|49472820 | NM_001539.2 | dnaJ homolog subfamily A member 1 [Homo sapiens] |
| 951 | gi\|62388891 | NM_002266.2 | importin subunit alpha-2 [Homo sapiens] |
| 952 | gi\|194440683 | NM_002337.2 | alpha-2-macroglobulin receptor-associated protein precursor [Homo sapiens] |
| 953 | gi\|93141028 | NM_001040134.1 | paralemmin isoform 2 [Homo sapiens] |
| 954 | gi\|103485495 | NM_002768.2 | charged multivesicular body protein 1a isoform 2 [Homo sapiens] |
| 955 | gi\|313760623 | NM_000442.4 | platelet endothelial cell adhesion molecule precursor [Homo sapiens] |
| 956 | gi\|47132588 | NM_002741.3 | serine/threonine-protein kinase N1 isoform 2 [Homo sapiens] |
| 957 | gi\|25777601 | NM_002808.3 | 26S proteasome non-ATPase regulatory subunit 2 [Homo sapiens] |
| 958 | gi\|30581139 | NM_006263.2 | proteasome activator complex subunit 1 isoform 1 [Homo sapiens] |
| 959 | gi\|104487294 | NM_130853.2 | receptor-type tyrosine-protein phosphatase S isoform 3 precursor [Homo sapiens] |
| 960 | gi\|71772428 | NM_001021.3 | 40S ribosomal protein S17 [Homo sapiens] |
| 961 | gi\|156416002 | NM_004168.2 | succinate dehydrogenase [ubiquinone] flavoprotein subunit, mitochondrial precursor [Homo sapiens] |
| 962 | gi\|118582263 | NM_006924.4 | serine/arginine-rich splicing factor 1 isoform 1 [Homo sapiens] |
| 963 | gi\|38181628 | BC061586.1 | TMSB4X protein [Homo sapiens] |
| 964 | gi\|37594440 | NM_003437.2 | zinc finger protein 136 [Homo sapiens] |
| 965 | gi\|83281439 | NM_003757.2 | eukaryotic translation initiation factor 3 subunit I [Homo sapiens] |
| 966 | gi\|23397695 | NM_152925.1 | copine-1 isoform a [Homo sapiens] |
| 967 | gi\|153791496 | NM_014675.3 | rootletin [Homo sapiens] |
| 968 | gi\|299829176 | NM_006989.5 | ras GTPase-activating protein 4 isoform 1 [Homo sapiens] |
| 969 | gi\|221307564 | NM_005805.4 | 26S proteasome non-ATPase regulatory subunit 14 [Homo sapiens] |
| 970 | gi\|138175804 | NM_006334.3 | noelin isoform 2 [Homo sapiens] |
| 971 | gi\|142379276 | NM_006541.3 | glutaredoxin-3 [Homo sapiens] |
| 972 | gi\|40805821 | NM_007263.3 | coatomer subunit epsilon isoform a [Homo sapiens] |
| 973 | gi\|254911095 | NM_001042486.2 | disks large-associated protein 4 isoform c [Homo sapiens] |
| 974 | gi\|194328807 | NM_012267.4 | hsp70-binding protein 1 [Homo sapiens] |
| 975 | gi\|42716281 | NM_013242.2 | transcription factor IIB [Homo sapiens] |
| 976 | gi\|54792141 | NM_019845.2 | protein reprimo [Homo sapiens] |
| 977 | gi\|209915598 | NM_032853.3 | PWWP domain-containing protein MUM1 [Homo sapiens] |
| 978 | gi\|50086623 | NM_033547.3 | integrator complex subunit 4 [Homo sapiens] |

**Tabelle B: Daten zu Markersequenzen für gynäkologisches Malignom (Aminosäure, Proteine, Peptide)SEQ ID No. 979 - 1497.**

| SEQ ID No. | Accession No. | Alias Accession No. | Blast |
|---|---|---|---|
| 979 | gi\|122114641 | NP_116313.3 | zinc finger protein 3 isoform 2 [Homo sapiens] |
| 980 | gi\|27881506 | NP_009120.1 | ATP-binding cassette sub-family F member 2 isoform a [Homo sapiens] |
| 981 | gi\|32880203 | NP_659422.2 | cadherin-24 isoform 2 [Homo sapiens] |
| 982 | gi\|261337183 | NP_878908.4 | WAS protein family homolog 1 [Homo sapiens] |
| 983 | gi\|16877438 | AAH16965.1 | NLRP1 protein [Homo sapiens] |
| 984 | gi\|16332364 | NP_277024.1 | cyclin-dependent kinase 11B isoform 5 [Homo sapiens] |
| 985 | gi\|4505715 | NP_002608.1 | peroxisome biogenesis factor 10 isoform 2 [Homo sapiens] |
| 986 | gi\|51477700 | NP_001003891.1 | mediator of RNA polymerase II transcription subunit 15 isoform a [Homo sapiens] |
| 987 | gi\|8923946 | NP_061137.1 | sphingomyelin phosphodiesterase 3 [Homo sapiens] |
| 988 | gi\|39644879 | AAH09967.2 | SCYL1 protein [Homo sapiens] |
| 989 | gi\|19718769 | NP_573403.1 | engulfment and cell motility protein 2 [Homo sapiens] |
| 990 | gi\|145553976 | NP_078933.3 | MORC family CW-type zinc finger protein 4 isoform a [Homo sapiens] |
| 991 | gi\|45387949 | NP_588609.1 | RING finger and SPRY domain-containing protein 1 precursor [Homo sapiens] |
| 992 | gi\|44681484 | NP_689775.2 | cell division cycle-associated protein 2 [Homo sapiens] |
| 993 | gi\|169216541 | XP_001717245.1 | PREDICTED: hypothetical protein [Homo sapiens] |
| 994 | gi\|50949425 | CAH10643.1 | hypothetical protein [Homo sapiens] |
| 995 | gi\|55925574 | NP_981949.1 | AMP deaminase 2 isoform 3 [Homo sapiens] |
| 996 | gi\|7020063 | BAA90981.1 | unnamed protein product [Homo sapiens] |
| 997 | gi\|41352718 | NP_004407.2 | protein deltex-1 [Homo sapiens] |
| 998 | gi\|4506141 | NP_002766.1 | serine protease HTRA1 precursor [Homo sapiens] |
| 999 | gi\|22027612 | NP_066961.2 | TNF receptor-associated factor 2 [Homo sapiens] |
| 1000 | gi\|4557873 | NP_000366.1 | uroporphyrinogen-III synthase [Homo sapiens] |
| 1001 | gi\|24307875 | NP_008886.1 | zinc finger protein 33B [Homo sapiens] |
| 1002 | gi\|4505971 | NP_003618.1 | large neutral amino acids transporter small subunit 3 [Homo sapiens] |
| 1003 | gi\|7657522 | NP_055060.1 | RING-box protein 2 isoform 1 [Homo sapiens] |
| 1004 | gi\|22748877 | NP_689627.1 | class E basic helix-loop-helix protein 22 [Homo sapiens] |
| 1005 | gi\|154759283 | NP_055418.2 | cerebellar degeneration-related protein 2-like [Homo sapiens] |
| 1006 | gi\|156415994 | NP_057121.2 | probable U3 small nucleolar RNA-associated protein 11 [Homo sapiens] |
| 1007 | gi\|282847378 | NP_542159.3 | apoptosis-related protein 3 isoform b [Homo sapiens] |
| 1008 | gi\|58331111 | NP_061919.1 | ankyrin repeat domain-containing protein 16 isoform a [Homo sapiens] |
| 1009 | gi\|54607104 | NP_064572.2 | chromosome 3 open reading frame 37 [Homo sapiens] |
| 1010 | gi\|57013272 | NP_001008860.1 | magnesium transporter NIPA2 isoform a [Homo sapiens] |
| 1011 | gi\|55770872 | NP_001007103.1 | lethal(3)malignant brain tumor-like protein 3 isoform b [Homo sapiens] |
| 1012 | gi\|24308354 | NP_149978.1 | hypothetical protein LOC89927 isoform 1 [Homo sapiens] |
| 1013 | gi\|37537684 | NP_009070.2 | zinc finger protein 92 isoform 1 [Homo sapiens] |
| 1014 | gi\|62988361 | NP_001615.1 | absent in melanoma 1 protein [Homo sapiens] |
| 1015 | gi\|4758472 | NP_004480.1 | G protein pathway suppressor 2 [Homo sapiens] |
| 1016 | gi\|18765733 | NP_003072.2 | synaptosomal-associated protein 25 isoform SNAP25A [Homo sapiens] |
| 1017 | gi\|6005920 | NP_009048.1 | prothyroliberin precursor [Homo sapiens] |
| 1018 | gi\|164519061 | NP_055505.3 | hypothetical protein LOC9715 isoform b [Homo sapiens] |
| 1019 | gi\|291327487 | NP_963863.2 | protein SMG7 isoform 4 [Homo sapiens] |
| 1020 | gi\|\|40787999 | NP_036578.2 | single-stranded DNA-binding protein 2 [Homo sapiens] |
| 1021 | gi\|28416940 | NP_057122.2 | ribosome maturation protein SBDS [Homo sapiens] |
| 1022 | gi\|11034821 | NP_067039.1 | endothelial zinc finger protein induced by tumor necrosis factor alpha [Homo sapiens] |
| 1023 | gi\|169636418 | NP_115867.2 | 39S ribosomal protein L38, mitochondrial precursor [Homo sapiens] |
| 1024 | gi\|186910286 | NP_078846.2 | ribonuclease H2 subunit B isoform 1 [Homo sapiens] |
| 1025 | gi\|87298935 | NP_078988.2 | engulfment and cell motility protein 3 [Homo sapiens] |
| 1026 | gi\|300068964 | NP_112484.3 | putative hydroxypyruvate isomerase isoform 1 [Homo sapiens] |
| 1027 | gi\|14150169 | NP_115736.1 | acyl-CoA-binding domain-containing protein 6 [Homo sapiens] |
| 1028 | gi\|119943096 | NP_115740.5 | dnaJ homolog subfamily C member 14 [Homo sapiens] |
| 1029 | gi\|333805596 | NP_689865.2 | zinc finger protein 48 isoform 1 [Homo sapiens] |
| 1030 | gi\|18375501 | NP_001632.2 | DNA-(apurinic or apyrimidinic site) lyase [Homo sapiens] |
| 1031 | gi\|214010181 | NP_001135748.1 | amyloid-like protein 2 isoform 2 [Homo sapiens] |
| 1032 | gi\|104487002 | NP_001035802.1 | receptor-type tyrosine-protein phosphatase delta isoform 5 precursor [Homo sapiens] |
| 1033 | gi\|32528306 | NP_002904.3 | replication factor C subunit 1 isoform 1 [Homo sapiens] |
| 1034 | gi\|41281489 | NP_055576.2 | IST1 homolog [Homo sapiens] |
| 1035 | gi\|193785998 | BAG50974.1 | unnamed protein product [Homo sapiens] |
| 1036 | gi\|217330576 | NP_009156.2 | neurexophilin-3 precursor [Homo sapiens] |
| 1037 | gi\|163310749 | NP_001106191.1 | LIM and calponin homology domains-containing protein 1 isoform e [Homo sapiens] |
| 1038 | gi\|38424073 | NP_055972.1 | pleckstrin homology-like domain family B member 1 isoform a [Homo sapiens] |
| 1039 | gi\|150456424 | NP_056163.1 | transmembrane protein 131 [Homo sapiens] |
| 1040 | gi\|11993943 | NP_055419.1 | tax1-binding protein 3 isoform 1 [Homo sapiens] |
| 1041 | gi\|66393093 | AAY45890.1 | phospholipase C epsilon 1 [Homo sapiens] |
| 1042 | gi\|55741655 | NP_066010.1 | anoctamin-8 [Homo sapiens] |
| 1043 | gi\|10863995 | NP_067011.1 | zinc finger protein 410 isoform b [Homo sapiens] |
| 1044 | gi\|205277445 | NP_068577.2 | single Ig IL-1-related receptor [Homo sapiens] |
| 1045 | gi\|205360981 | NP_078833.3 | protein RIC-3 isoform a [Homo sapiens] |
| 1046 | gi\|29789255 | NP_085132.1 | C-Maf-inducing protein isoform Tc-mip [Homo sapiens] |
| 1047 | gi\|14502023 | NP_001617.1 | RAC-beta serine/threonine-protein kinase isoform 1 [Homo sapiens] |
| 1048 | gi\|34577049 | NP_056363.2 | bullous pemphigoid antigen 1 isoform 1eA precursor [Homo sapiens] |
| 1049 | gi\|4826686 | NP_004930.1 | ATP-dependent RNA helicase DDX1 [Homo sapiens] |
| 1050 | gi\|4503979 | NP_002046.1 | glial fibrillary acidic protein isoform 1 [Homo sapiens] |
| 1051 | gi\|156104891 | NP_002087.2 | general transcription factor IIF subunit 1 [Homo sapiens] |
| 1052 | gi\|167466173 | NP_005337.2 | heat shock 70 kDa protein 1A/1B [Homo sapiens] |
| 1053 | gi\|4506439 | NP_002884.1 | histone-binding protein RBBP7 isoform 2 [Homo sapiens] |
| 1054 | gi\|18375673 | NP_002902.2 | regulator of nonsense transcripts 1 [Homo sapiens] |
| 1055 | gi\|218083730 | NP_001136156.1 | zinc finger MYM-type protein 5 isoform 3 [Homo sapiens] |
| 1056 | gi\|5729828 | NP_006691.1 | TRAF-type zinc finger domain-containing protein 1 [Homo sapiens] |
| 1057 | gi\|6857826 | NP_006702.1 | RNA-binding protein with serine-rich domain 1 [Homo sapiens] |
| 1058 | gi\|5902134 | NP_009005.1 | coronin-1A [Homo sapiens] |
| 1059 | gi\|7705819 | NP_057181.1 | immediate early response 3-interacting protein 1 [Homo sapiens] |
| 1060 | gi\|8923896 | NP_061147.1 | acetyl-coenzyme A synthetase, cytoplasmic isoform 1 [Homo sapiens] |
| 1061 | gi\|51873031 | NP_064555.2 | nicalin precursor [Homo sapiens] |
| 1062 | gi\|116174742 | NP_064608.2 | dentin matrix protein 4 [Homo sapiens] |
| 1063 | gi\|187761324 | NP_001120683.1 | shootin-1 isoform a [Homo sapiens] |
| 1064 | gi\|38372935 | NP_068767.3 | brevican core protein isoform 1 [Homo sapiens] |
| 1065 | gi\|150378520 | NP_115919.1 | zinc finger protein 594 [Homo sapiens] |
| 1066 | gi\|81174549 | NP_001032240.1 | STAT3-interacting protein as a repressor [Homo sapiens] |
| 1067 | gi\|83921602 | NP_001033073.1 | putative sodium-coupled neutral amino acid transporter 10 isoform a [Homo sapiens] |
| 1068 | gi\|222831660 | NP_940877.2 | hypothetical protein LOC284069 precursor [Homo sapiens] |
| 1069 | gi\|37620163 | NP_065741.3 | ANKHD1-EIF4EBP3 protein [Homo sapiens] |
| 1070 | gi\|166362737 | NP_001107606.1 | erythrocyte membrane protein band 4.2 isoform 2 [Homo sapiens] |
| 1071 | gi\|51896035 | NP_002073.2 | G protein-coupled receptor kinase 6 isoform B [Homo sapiens] |
| 1072 | gi\|6006001 | NP_002075.2 | glutathione peroxidase 3 precursor [Homo sapiens] |
| 1073 | gi\|4758786 | NP_004541.1 | NADH dehydrogenase [ubiquinone] iron-sulfur protein 2, |
| | | | mitochondrial isoform 1 precursor [Homo sapiens] |
| 1074 | gi\|23110925 | NP_002789.1 | proteasome subunit beta type-6 precursor [Homo sapiens] |
| 1075 | gi\|22094079 | NP_004764.1 | zinc finger HIT domain-containing protein 3 [Homo sapiens] |
| 1076 | gi\|4759274 | NP_004777.1 | thioredoxin-like protein 1 [Homo sapiens] |
| 1077 | gi\|5729889 | NP_006685.1 | protein JTB precursor [Homo sapiens] |
| 1078 | gi\|6996928 | NP_006783.2 | mortality factor 4 [Homo sapiens] |
| 1079 | gi\|28373192 | NP_008933.2 | proteasomal ubiquitin receptor ADRM1 precursor [Homo sapiens] |
| 1080 | gi\|33188445 | NP_036222.3 | microtubule-actin cross-linking factor 1 isoform a [Homo sapiens] |
| 1081 | gi\|13489112 | NP_055153.1 | phosphatidylserine decarboxylase proenzyme [Homo sapiens] |
| 1082 | gi\|24308115 | NP_056464.1 | interferon regulatory factor 2-binding protein 1 [Homo sapiens] |
| 1083 | gi\|115430235 | NP_001041666.1 | E3 ubiquitin-protein ligase UHRF1 isoform 1 [Homo sapiens] |
| 1084 | gi\|56676371 | NP_037423.2 | cleavage and polyadenylation specificity factor subunit 1 [Homo sapiens] |
| 1085 | gi\|7706712 | NP_057622.1 | NAD-dependent deacetylase sirtuin-7 [Homo sapiens] |
| 1086 | gi\|16554604 | NP_057154.2 | 28S ribosomal protein S23, mitochondrial [Homo sapiens] |
| 1087 | gi\|11545918 | NP_071447.1 | tubulointerstitial nephritis antigen-like isoform 1 precursor [Homo sapiens] |
| 1088 | gi\|282400942 | NP_073574.2 | lipid phosphate phosphatase-related protein type 2 isoform 1 [Homo sapiens] |
| 1089 | gi\|14150171 | NP_115737.1 | THO complex subunit 3 [Homo sapiens] |
| 1090 | gi\|34304354 | NP_899067.1 | phosphatase and actin regulator 3 isoform 2 [Homo sapiens] |
| 1091 | gi\|194380674 | BAG58490.1 | unnamed protein product [Homo sapiens] |
| 1092 | gi\|66882524 | NP_001018196.1 | protein farnesyltransferase/geranylgeranyltransferase type-1 subunit alpha isoform b [Homo sapiens] |
| 1093 | gi\|38788319 | NP_005266.2 | guanine nucleotide-binding protein-like 1 [Homo sapiens] |
| 1094 | gi\|11321591 | NP_002120.1 | high mobility group protein B2 [Homo sapiens] |
| 1095 | gi\|156119625 | NP_002206.2 | inter-alpha-trypsin inhibitor heavy chain H1 isoform a [Homo sapiens] |
| 1096 | gi\|167614504 | NP_002282.2 | laminin subunit beta-1 precursor [Homo sapiens] |
| 1097 | gi\|205277383 | NP_066278.3 | hepatocyte growth factor-like protein precursor [Homo sapiens] |
| 1098 | gi\|24430151 | NP_002793.2 | 26S protease regulatory subunit 4 [Homo sapiens] |
| 1099 | gi\|5803187 | NP_006746.1 | transaldolase [Homo sapiens] |
| 1100 | gi\|4508029 | NP_003416.1 | zinc finger protein 45 [Homo sapiens] |
| 1101 | gi\|14506399 | NP_003601.1 | mRNA export factor [Homo sapiens] |
| 1102 | gi\|109452591 | NP_003840.2 | succinyl-CoA ligase [GDP-forming] subunit alpha, mitochondrial precursor [Homo sapiens] |
| 1103 | gi\|32171175 | NP_003901.2 | protein BUD31 homolog [Homo sapiens] |
| 1104 | gi\|117938254 | NP_001070909.1 | bcl-2-associated transcription factor 1 isoform 3 [Homo sapiens] |
| 1105 | gi\|18765702 | NP_569709.1 | engulfment and cell motility protein 1 isoform 2 [Homo sapiens] |
| 1106 | gi\|10863909 | NP_066925.1 | kunitz-type protease inhibitor 2 isoform a precursor [Homo sapiens] |
| 1107 | gi\|4884171 | CAB43208.1 | hypothetical protein [Homo sapiens] |
| 1108 | gi\|194385126 | BAG60969.1 | unnamed protein product [Homo sapiens] |
| 1109 | gi\|15147337 | NP_056986.2 | E3 ubiquitin-protein ligase UBR5 [Homo sapiens] |
| 1110 | gi\|22035562 | NP_060780.2 | transcription factor IIIB 50 kDa subunit [Homo sapiens] |
| 1111 | gi\|122939167 | NP_062559.2 | WD repeat domain phosphoinositide-interacting protein 3 [Homo sapiens] |
| 1112 | gi\|151301035 | NP_064536.2 | stAR-related lipid transfer protein 7, mitochondrial precursor [Homo sapiens] |
| 1113 | gi\|13129110 | NP_077007.1 | methylosome protein 50 [Homo sapiens] |
| 1114 | gi\|75677353 | NP_114127.3 | ATPase family AAA domain-containing protein 3B [Homo sapiens] |
| 1115 | gi\|13994300 | NP_114131.1 | transmembrane protein 120A [Homo sapiens] |
| 1116 | gi\|14150054 | NP_115674.1 | synaptotagmin-3 [Homo sapiens] |
| 1117 | gi\|54112382 | NP_115979.3 | lysine-specific demethylase 2B isoform a [Homo sapiens] |
| 1118 | gi\|28372509 | NP_777568.1 | coiled-coil domain-containing protein 50 short isoform [Homo sapiens] |
| 1119 | gi\|14210524 | NP_115904.1 | bcl-2-related ovarian killer protein [Homo sapiens] |
| 1120 | gi\|4502491 | NP_001203.1 | complement component 1 Q subcomponent-binding protein, mitochondrial precursor [Homo sapiens] |
| 1121 | gi\|4557403 | NP_000378.1 | mitochondrial carnitine/acylcarnitine carrier protein [Homo sapiens] |
| 1122 | gi\|4758120 | NP_004385.1 | death-associated protein 1 [Homo sapiens] |
| 1123 | gi\|55956921 | NP_004490.2 | heterogeneous nuclear ribonucleoprotein A/B isoform b [Homo sapiens] |
| 1124 | gi\|62632771 | NP_001015054.1 | DNA-3-methyladenine glycosylase isoform c [Homo sapiens] |
| 1125 | gi\|76150623 | NP_006161.2 | putative ribosomal RNA methyltransferase NOP2 [Homo sapiens] |
| 1126 | gi\|4505641 | NP_002583.1 | proliferating cell nuclear antigen [Homo sapiens] |
| 1127 | gi\|4507761 | NP_003324.1 | ubiquitin-60S ribosomal protein L40 precursor [Homo sapiens] |
| 1128 | gi\|190684675 | NP_006288.2 | DNA repair protein XRCC1 [Homo sapiens] |
| 1129 | gi\|52630423 | NP_003572.2 | cytoplasmic protein NCK2 isoform A [Homo sapiens] |
| 1130 | gi\|4506753 | NP_003698.1 | ruvB-like 1 [Homo sapiens] |
| 1131 | gi\|4758808 | NP_004832.1 | ras GTPase-activating protein nGAP isoform 1 [Homo sapiens] |
| 1132 | gi\|4758540 | NP_004845.1 | carbohydrate sulfotransferase 10 [Homo sapiens] |
| 1133 | gi\|259013556 | NP_004851.2 | fragile X mental retardation syndrome-related protein 2 [Homo sapiens] |
| 1134 | gi\|53729352 | NP_005103.2 | WD repeat-containing protein 1 isoform 2 [Homo sapiens] |
| 1135 | gi\|6807759 | CAB70684.1 | hypothetical protein [Homo sapiens] |
| 1136 | gi\|14149702 | NP_056343.1 | E3 ubiquitin-protein ligase RNF167 precursor [Homo sapiens] |
| 1137 | gi\|24430139 | NP_056355.2 | RNA polymerase II-associated protein 1 [Homo sapiens] |
| 1138 | gi\|15193488 | NP_055232.1 | bcl-2-binding component 3 isoform 4 [Homo sapiens] |
| 1139 | gi\|22547136 | NP_057040.2 | 39S ribosomal protein L4, mitochondrial isoform a [Homo sapiens] |
| 1140 | gi\|7657015 | NP_055121.1 | tRNA-splicing ligase RtcB homolog [Homo sapiens] |
| 1141 | gi\|41055953 | NP_060688.1 | ubiquitin carboxyl-terminal hydrolase 40 [Homo sapiens] |
| 1142 | gi\|145553959 | NP_060597.4 | zinc phosphodiesterase ELAC protein 2 isoform 1 [Homo sapiens] |
| 1143 | gi\|1242247075 | NP_001009877.2 | bromodomain-containing protein 9 isoform 2 [Homo sapiens] |
| 1144 | gi\|110349769 | NP_077272.2 | coiled-coil domain-containing protein 28B [Homo sapiens] |
| 1145 | gi\|13236585 | NP_077309.1 | fibronectin type III and SPRY domain-containing protein 1 [Homo sapiens] |
| 1146 | gi\|33337996 | AAQ13620.1 | MSTP107 [Homo sapiens] |
| 1147 | gi\|31377667 | NP_113678.2 | Ion protease homolog 2, peroxisomal [Homo sapiens] |
| 1148 | gi\|206597513 | NP_056057.2 | arf-GAP with Rho-GAP domain, ANK repeat and PH domain-containing protein 1 isoform a [Homo sapiens] |
| 1149 | gi\|145387955 | NP_991330.1 | protein QIL1 [Homo sapiens] |
| 1150 | gi\|134288890 | NP_689596.4 | DIS3-like exonuclease 2 [Homo sapiens] |
| 1151 | gi\|42794622 | NP_976308.1 | zinc finger protein 784 [Homo sapiens] |
| 1152 | gi\|113424119 | XP_370711.4 | PREDICTED: similar to FRAS1 related extracellular matrix protein 2 [Homo sapiens] |
| 1153 | gi\|4503285 | NP_001345.1 | aldo-keto reductase family 1 member C2 isoform 1 [Homo sapiens] |
| 1154 | gi\|100913206 | NP_001348.2 | ATP-dependent RNA helicase A [Homo sapiens] |
| 1155 | gi\|22091454 | NP_665683.1 | glutathione S-transferase A1 [Homo sapiens] |
| 1156 | gi\|15626999 | NP_258412.1 | inosine triphosphate pyrophosphatase isoform a [Homo sapiens] |
| 1157 | gi\|14702171 | NP_116558.1 | DNA-directed RNA polymerase II subunit RPB3 [Homo sapiens] |
| 1158 | gi\|110618253 | NP_005026.3 | DNA-directed RNA polymerase, mitochondrial precursor [Homo sapiens] |
| 1159 | gi\|21361547 | NP_002930.2 | ribonuclease inhibitor [Homo sapiens] |
| 1160 | gi\|4506749 | NP_001024.1 | ribonucleoside-diphosphate reductase large subunit [Homo sapiens] |
| 1161 | gi\|71051616 | NP_000365.3 | uroporphyrinogen decarboxylase [Homo sapiens] |
| 1162 | gi\|22027642 | NP_036421.2 | kelch-like ECH-associated protein 1 [Homo sapiens] |
| 1163 | gi\|194294519 | NP_001123574.1 | oxysterols receptor LXR-alpha isoform c [Homo sapiens] |
| 1164 | gi\|7706337 | NP_057141.1 | coatomer subunit zeta-1 [Homo sapiens] |
| 1165 | gi 122114658 | NP_055852.2 | hypothetical protein LOC23053 isoform 1 [Homo sapiens] |
| 1166 | gi 150456432 | NP_001092906.1 | serine/threonine-protein kinase ULK3 [Homo sapiens] |
| 1167 | gi 18490987 | NP_057267.2 | mRNA turnover protein 4 homolog [Homo sapiens] |
| 1168 | gi 116812575 | NP_057342.2 | YTH domain family protein 2 isoform 1 [Homo sapiens] |
| 1169 | gi 14249156 | NP_116019.1 | cyclin-dependent kinase 2-interacting protein isoform 2 [Homo sapiens] |
| 1170 | gi\|62526026 | NP_001014840.1 | protein CutA isoform 3 [Homo sapiens] |
| 1171 | gi\|68533249 | NP_060946.3 | protein BEX1 [Homo sapiens] |
| 1172 | gi\|94818879 | NP_001035547.1 | rhomboid domain-containing protein 2 isoform b [Homo sapiens] |
| 1173 | gi\|83779010 | NP_076982.3 | selenocysteine insertion sequence-binding protein 2 [Homo sapiens] |
| 1174 | gi\|208431769 | NP_113638.2 | basophilic leukemia expressed protein BLES03 isoform 2 [Homo sapiens] |
| 1175 | gi\|13899354 | NP_113680.1 | RNA-binding protein 4B [Homo sapiens] |
| 1176 | gi\|20270317 | NP_620132.1 | ribosome-recycling factor, mitochondrial isoform 1 precursor [Homo sapiens] |
| 1177 | gi\|23397663 | NP_612213.2 | abhydrolase domain-containing protein 3 [Homo sapiens] |
| 1178 | gi\|23510323 | NP_055917.1 | nephrocystin-4 [Homo sapiens] |
| 1179 | gi\|49169841 | NP_001001795.1 | hypothetical protein LOC414919 [Homo sapiens] |
| 1180 | gi\|9257192 | NP_001608.1 | beta-adducin isoform a [Homo sapiens] |
| 1181 | gi\|148539876 | NP_001610.2 | beta-adrenergic receptor kinase 1 [Homo sapiens] |
| 1182 | gi\|4557367 | NP_000377.1 | bleomycin hydrolase [Homo sapiens] |
| 1183 | gi\|55770844 | NP_001894.2 | catenin alpha-1 [Homo sapiens] |
| 1184 | gi\|40288197 | NP_005248.2 | transcription factor GATA-6 [Homo sapiens] |
| 1185 | gi\|4885371 | NP_005309.1 | histone H1.0 [Homo sapiens] |
| 1186 | gi\|4885419 | NP_005332.1 | zinc finger and BTB domain-containing protein 48 [Homo sapiens] |
| 1187 | gi\|4504505 | NP_000405.1 | peroxisomal multifunctional enzyme type 2 isoform 2 [Homo sapiens] |
| 1188 | gi\|33356547 | NP_004517.2 | DNA replication licensing factor MCM2 [Homo sapiens] |
| 1189 | gi\|29826282 | NP_817092.1 | protein phosphatase 1G [Homo sapiens] |
| 1190 | gi\|4506609 | NP_000972.1 | 60S ribosomal protein L19 [Homo sapiens] |
| 1191 | gi\|4826998 | NP_005057.1 | splicing factor, proline- and glutamine-rich [Homo sapiens] |
| 1192 | gi\|86991438 | NP_001034554.1 | serine/arginine-rich splicing factor 5 [Homo sapiens] |
| 1193 | gi\|170014703 | NP_003014.3 | SH3 domain-binding protein 2 isoform a [Homo sapiens] |
| 1194 | gi\|20127452 | NP_003241.2 | thyroid hormone receptor alpha isoform 2 [Homo sapiens] |
| 1195 | gi\|5730091 | NP_006519.1 | tissue factor pathway inhibitor 2 precursor [Homo sapiens] |
| 1196 | gi\|4885487 | NP_005430.1 | myeloid leukemia factor 2 [Homo sapiens] |
| 1197 | gi\|201860300 | NP_003968.2 | AH receptor-interacting protein [Homo sapiens] |
| 1198 | gi\|55741719 | NP_055621.1 | iporin [Homo sapiens] |
| 1199 | gi\|124378039 | NP_055681.1 | protein transport protein Sec16A [Homo sapiens] |
| 1200 | gi\|23308699 | NP_005452.2 | transcription factor MafB [Homo sapiens] |
| 1201 | gi\|5454048 | NP_006370.1 | semaphorin-3C precursor [Homo sapiens] |
| 1202 | gi\|32454737 | NP_150594.2 | tripartite motif-containing protein 3 [Homo sapiens] |
| 1203 | gi\|116256487 | NP_006693.3 | neuropathy target esterase isoform b [Homo sapiens] |
| 1204 | gi\|21758256 | BAC05274.1 | unnamed protein product [Homo sapiens] |
| 1205 | gi\|6912328 | NP_036269.1 | N(G),N(G)-dimethylarginine dimethylaminohydrolase 1 isoform 1 [Homo sapiens] |
| 1206 | gi\|223468620 | NP_038474.2 | E3 ubiquitin-protein ligase makorin-1 isoform 1 [Homo sapiens] |
| 1207 | gi\|11385648 | AAG34905.1 | CTCL tumor antigen se14-3 [Homo sapiens] |
| 1208 | gi\|196115158 | NP_001124490.1 | cip1-interacting zinc finger protein isoform 4 [Homo sapiens] |
| 1209 | gi\|29648315 | NP_056520.2 | small G protein signaling modulator 3 [Homo sapiens] |
| 1210 | gi\|7020525 | BAA91164.1 | unnamed protein product [Homo sapiens] |
| 1211 | gi\|217035105 | NP_060668.2 | dnaJ homolog subfamily C member 11 [Homo sapiens] |
| 1212 | gi\|13129000 | NP_076947.1 | meteorin precursor [Homo sapiens] |
| 1213 | gi\|117320543 | NP_001070965.1 | proline-rich protein 3 isoform b [Homo sapiens] |
| 1214 | gi\|38176285 | NP_115569.2 | ribonuclease H2 subunit C [Homo sapiens] |
| 1215 | gi\|20270303 | NP_620124.1 | mitochondrial Rho GTPase 2 [Homo sapiens] |
| 1216 | gi\|15529984 | NP_219483.1 | armadillo repeat-containing protein 6 isoform 2 [Homo sapiens] |
| 1217 | gi\|62244044 | NP_001014446.1 | OCIA domain-containing protein 2 isoform 1 [Homo sapiens] |
| 1218 | gi\|333609248 | NP_695001.2 | hypothetical protein LOC140680 isoform 1 [Homo sapiens] |
| 1219 | gi\|21389515 | NP_653327.1 | RING finger protein 145 isoform 2 [Homo sapiens] |
| 1220 | gi\|5453579 | NP_006120.1 | bone morphogenetic protein 1 isoform 3 precursor [Homo sapiens] |
| 1221 | gi\|169790899 | NP_001116102.1 | cyclin-dependent kinase inhibitor 1C isoform b [Homo sapiens] |
| 1222 | gi\|4503139 | NP_001899.1 | cathepsin B preproprotein [Homo sapiens] |
| 1223 | gi\|38201714 | NP_001410.2 | ELAV-like protein 1 [Homo sapiens] |
| 1224 | gi\|20070199 | NP_005225.2 | nuclear receptor subfamily 2 group F member 6 [Homo sapiens] |
| 1225 | gi\|11497612 | NP_068703.1 | gamma-aminobutyric acid type B receptor subunit 1 isoform b precursor [Homo sapiens] |
| 1226 | gi\|27502383 | NP_758527.1 | homeobox protein Meis2 isoform h [Homo sapiens] |
| 1227 | gi\|4826860 | NP_004999.1 | NHP2-like protein 1 [Homo sapiens] |
| 1228 | gi\|20072757 | AAH27470.1 | PCDHGC3 protein [Homo sapiens] |
| 1229 | gi\|25777612 | NP_002800.2 | 26S proteasome non-ATPase regulatory subunit 3 [Homo sapiens] |
| 1230 | gi\|18450369 | NP_543030.1 | receptor-type tyrosine-protein phosphatase alpha isoform 2 precursor [Homo sapiens] |
| 1231 | gi\|5454004 | NP_006259.1 | zinc finger protein ubi-d4 [Homo sapiens] |
| 1232 | gi\|6677723 | NP_031396.1 | replication factor C subunit 5 isoform 1 [Homo sapiens] |
| 1233 | gi\|4506635 | NP_000985.1 | 60S ribosomal protein L32 [Homo sapiens] |
| 1234 | gi\|4506929 | NP_003016.1 | endophilin-A2 isoform 1 [Homo sapiens] |
| 1235 | gi\|27764867 | NP_003170.1 | synaptophysin [Homo sapiens] |
| 1236 | gi\|49472822 | NP_003746.2 | eukaryotic translation initiation factor 3 subunit G [Homo sapiens] |
| 1237 | gi\|331284176 | NP_001070729.2 | nuclear receptor corepressor 2 isoform 2 [Homo sapiens] |
| 1238 | gi\|5031937 | NP_005822.1 | calcium-binding and coiled-coil domain-containing protein 2 [Homo sapiens] |
| 1239 | gi\|6005846 | NP_009215.1 | twinfilin-2 [Homo sapiens] |
| 1240 | gi\|48257171 | AAH26067.2 | WDSOF1 protein [Homo sapiens] |
| 1241 | gi\|155030236 | NP_001093882.1 | ribonuclease 3 isoform 2 [Homo sapiens] |
| 1242 | gi\|22547191 | NP_037438.2 | sorting nexin-15 isoform A [Homo sapiens] |
| 1243 | gi\|109134347 | NP_001035858.1 | coiled-coil domain-containing protein 41 [Homo sapiens] |
| 1244 | gi\|50409707 | NP_057722.2 | ADP-ribosylation factor-like protein 6-interacting protein 4 isoform 2 [Homo sapiens] |
| 1245 | gi\|7706757 | NP_057078.1 | V-type proton ATPase subunit D [Homo sapiens] |
| 1246 | gi\|85362737 | NP_001034229.1 | hypothetical protein LOC54976 [Homo sapiens] |
| 1247 | gi\|21750405 | BAC03773.1 | unnamed protein product [Homo sapiens] |
| 1248 | gi\|157388941 | NP_060468.2 | hypothetical protein LOC55071 [Homo sapiens] |
| 1249 | gi\|122937245 | NP_001073894.1 | protein BEX4 [Homo sapiens] |
| 1250 | gi\|46559761 | NP_115901.2 | PDZ domain-containing protein 4 [Homo sapiens] |
| 1251 | gi\|198041728 | NP_001128246.1 | kinesin light chain 2 isoform 2 [Homo sapiens] |
| 1252 | gi\|55749442 | NP_001006936.1 | transcription elongation factor A protein-like 4 [Homo sapiens] |
| 1253 | gi\|18027792 | AAL55857.1 | unknown [Homo sapiens] |
| 1254 | gi\|116235476 | NP_116245.2 | WD repeat-containing protein 73 [Homo sapiens] |
| 1255 | gi\|46195763 | NP_612392.1 | MTSS1-like protein [Homo sapiens] |
| 1256 | gi\|156523246 | NP_660282.2 | proline-rich transmembrane protein 2 [Homo sapiens] |
| 1257 | gi\|32698884 | NP_872333.1 | calcium-binding protein 7 [Homo sapiens] |
| 1258 | gi\|195947382 | NP_847884.2 | RILP-like protein 1 [Homo sapiens] |
| 1259 | gi\|4757768 | NP_004300.1 | rho GDP-dissociation inhibitor 1 isoform a [Homo sapiens] |
| 1260 | gi\|4503143 | NP_001900.1 | cathepsin D preproprotein [Homo sapiens] |
| 1261 | gi\|12669911 | NP_005216.1 | transcription factor E2F1 [Homo sapiens] |
| 1262 | gi\|188497703 | NP_004177.3 | semaphorin-3F precursor [Homo sapiens] |
| 1263 | gi\|109150416 | NP_036425.1 | peroxidasin homolog precursor [Homo sapiens] |
| 1264 | gi\|115430229 | NP_005123.2 | meiotic recombination protein REC8 homolog [Homo sapiens] |
| 1265 | gi\|5174391 | NP_006057.1 | alcohol dehydrogenase [NADP+] [Homo sapiens] |
| 1266 | gi\|5453838 | NP_006387.1 | Sjoegren syndrome/scleroderma autoantigen 1 [Homo sapiens] |
| 1267 | gi\|6005794 | NP_009144.1 | PRA1 family protein 2 [Homo sapiens] |
| 1268 | gi\|6912674 | NP_036569.1 | SNARE-associated protein Snapin [Homo sapiens] |
| 1269 | gi\|148664190 | NP_055148.3 | cell adhesion molecule 1 isoform 1 [Homo sapiens] |
| 1270 | gi\|18496983 | NP_056341.1 | CAP-Gly domain-containing linker protein 3 [Homo sapiens] |
| 1271 | gi\|9257240 | NP_059429.1 | neuroplastin isoform a precursor [Homo sapiens] |
| 1272 | gi\|116812573 | NP_057487.2 | protein CWC15 homolog [Homo sapiens] |
| 1273 | gi\|10438636 | BAB15296.1 | unnamed protein product [Homo sapiens] |
| 1274 | gi\|24429588 | NP_060651.2 | zinc finger protein 532 [Homo sapiens] |
| 1275 | gi\|8922735 | NP_060725.1 | elongator complex protein 2 isoform 2 [Homo sapiens] |
| 1276 | gi\|18034767 | NP_071387.1 | phosphorylated CTD-interacting factor 1 [Homo sapiens] |
| 1277 | gi\|40068485 | NP_073745.2 | von Willebrand factor A domain-containing protein 1 isoform 1 precursor [Homo sapiens] |
| 1278 | gi\|215820630 | NP_115731.2 | vacuolar fusion protein MON1 homolog A isoform a [Homo sapiens] |
| 1279 | gi\|87196600 | NP_001034592.1 | serine protease 53 precursor [Homo sapiens] |
| 1280 | gi\|24307879 | NP_001369.1 | cytoplasmic dynein 1 intermediate chain 2 [Homo sapiens] |
| 1281 | gi\|153791535 | NP_066564.2 | histone acetyltransferase KAT2A [Homo sapiens] |
| 1282 | gi\|73747844 | NP_002302.2 | DNA ligase 3 isoform beta precursor [Homo sapiens] |
| 1283 | gi\|4557797 | NP_000260.1 | nucleoside diphosphate kinase A isoform b [Homo sapiens] |
| 1284 | gi\|14505947 | NP_002687.1 | DNA-directed RNA polymerase II subunit RPB7 [Homo sapiens] |
| 1285 | gi\|56790299 | NP_002770.3 | PH and SEC7 domain-containing protein 1 [Homo sapiens] |
| 1286 | gi\|21359854 | NP_004573.2 | geranylgeranyl transferase type-2 subunit beta [Homo sapiens] |
| 1287 | gi\|4507709 | NP_003304.1 | GDP-L-fucose synthase [Homo sapiens] |
| 1288 | gi\|14507725 | NP_000362.1 | transthyretin precursor [Homo sapiens] |
| 1289 | gi\|193211616 | NP_003625.2 | protein NipSnap homolog 1 isoform 1 [Homo sapiens] |
| 1290 | gi\|21396489 | NP_004784.2 | Ion protease homolog, mitochondrial precursor [Homo sapiens] |
| 1291 | gi\|4885495 | NP_005485.1 | dnaJ homolog subfamily B member 6 isoform b [Homo sapiens] |
| 1292 | gi\|18491024 | NP_005691.2 | dipeptidyl peptidase 3 [Homo sapiens] |
| 1293 | gi\|5729781 | NP_006642.1 | complexin-1 [Homo sapiens] |
| 1294 | gi\|25777682 | NP_733831.1 | ras association domain-containing protein 1 isoform C [Homo sapiens] |
| 1295 | gi\|114796626 | NP_037488.2 | monocarboxylate transporter 3 [Homo sapiens] |
| 1296 | gi\|24430132 | NP_036610.2 | WW domain-binding protein 2 [Homo sapiens] |
| 1297 | gi\|9506931 | NP_061955.1 | probable ATP-dependent RNA helicase DDX56 [Homo sapiens] |
| 1298 | gi\|33859678 | NP_060349.1 | zinc finger protein 416 [Homo sapiens] |
| 1299 | gi\|24432026 | NP_116164.2 | kelch-like protein 22 [Homo sapiens] |
| 1300 | gi\|116812612 | NP_653187.3 | ly6/PLAUR domain-containing protein 1 isoform a [Homo sapiens] |
| 1301 | gi\|40548332 | NP_954981.1 | coiled-coil domain-containing protein 137 [Homo sapiens] |
| 1302 | gi\|148763345 | NP_277071.2 | cyclin-dependent kinase 11A isoform 4 [Homo sapiens] |
| 1303 | gi\|38372925 | NP_940991.1 | basigin isoform 2 precursor [Homo sapiens] |
| 1304 | gi\|40804468 | NP_000714.3 | voltage-dependent L-type calcium channel subunit beta-1 isoform 1 [Homo sapiens] |
| 1305 | gi\|4503093 | NP_001885.1 | casein kinase I isoform epsilon [Homo sapiens] |
| 1306 | gi\|9845505 | NP_004143.1 | ornithine decarboxylase antizyme 1 [Homo sapiens] |
| 1307 | gi\|4506401 | NP_002871.1 | RAF proto-oncogene serine/threonine-protein kinase [Homo sapiens] |
| 1308 | gi\|4506741 | NP_001002.1 | 40S ribosomal protein S7 [Homo sapiens] |
| 1309 | gi\|4506679 | NP_001005.1 | 40S ribosomal protein S10 [Homo sapiens] |
| 1310 | gi\|48255970 | NP_002960.2 | mitogen-activated protein kinase 12 [Homo sapiens] |
| 1311 | gi\|56117850 | NP_009089.4 | cold shock domain-containing protein E1 isoform 2 [Homo sapiens] |
| 1312 | gi\|18375630 | NP_542433.1 | large proline-rich protein BAG6 isoform b [Homo sapiens] |
| 1313 | gi\|24234683 | NP_004642.2 | ubiquitin carboxyl-terminal hydrolase 11 [Homo sapiens] |
| 1314 | gi\|4507947 | NP_003671.1 | tyrosyl-tRNA synthetase, cytoplasmic [Homo sapiens] |
| 1315 | gi\|195972859 | NP_004219.3 | cytohesin-2 isoform 2 [Homo sapiens] |
| 1316 | gi\|5453567 | NP_006315.1 | craniofacial development protein 1 [Homo sapiens] |
| 1317 | gi\|16554627 | NP_060058.1 | WD repeat-containing protein 5 [Homo sapiens] |
| 1318 | gi\|6005854 | NP_009204.1 | prohibitin-2 isoform 2 [Homo sapiens] |
| 1319 | gi\|6005768 | NP_009216.1 | gamma-aminobutyric acid receptor-associated protein-like 2 [Homo sapiens] |
| 1320 | gi\|7019491 | NP_037406.1 | DNA polymerase lambda isoform a [Homo sapiens] |
| 1321 | gi\|21361670 | NP_054782.2 | drebrin-like protein isoform a [Homo sapiens] |
| 1322 | gi\|17978481 | NP_536338.1 | vacuolar protein sorting-associated protein 16 homolog isoform 3 [Homo sapiens] |
| 1323 | gi\|13027612 | NP_076429.1 | ras-like protein family member 11B [Homo sapiens] |
| 1324 | gi\|190194416 | NP_077302.3 | F-box/LRR-repeat protein 15 [Homo sapiens] |
| 1325 | gi\|16604252 | NP_443092.1 | zinc finger protein 300 isoform 2 [Homo sapiens] |
| 1326 | gi\|115511030 | NP_612450.2 | protein MB21D1 [Homo sapiens] |
| 1327 | gi\|23510413 | NP_443180.2 | rho GTPase-activating protein 33 isoform 1 [Homo sapiens] |
| 1328 | gi\|38788219 | NP_872304.2 | zinc finger protein 428 [Homo sapiens] |
| 1329 | gi\|58761496 | NP_001011724.1 | heterogeneous nuclear ribonucleoprotein A1-like 2 [Homo sapiens] |
| 1330 | gi\|34530769 | BAC85972.1 | unnamed protein product [Homo sapiens] |
| 1331 | gi\|4557010 | NP_001601.1 | lysosomal acid phosphatase isoform 1 precursor [Homo sapiens] |
| 1332 | gi\|12025678 | NP_004915.2 | alpha-actinin-4 [Homo sapiens] |
| 1333 | gi\|4502981 | NP_001852.1 | cytochrome c oxidase subunit 4 isoform 1, mitochondrial precursor [Homo sapiens] |
| 1334 | gi\|20357529 | NP_005264.2 | guanine nucleotide-binding protein G(I)/G(S)/G(T) subunit beta-2 [Homo sapiens] |
| 1335 | gi\|4885375 | NP_005310.1 | histone H1.2 [Homo sapiens] |
| 1336 | gi\|188497750 | NP_277035.2 | hexokinase-1 isoform HKI-td [Homo sapiens] |
| 1337 | gi\|250084 | AAA09035.1 | HSJ1b [Homo sapiens] |
| 1338 | gi\|4758756 | NP_004528.1 | nucleosome assembly protein 1-like 1 [Homo sapiens] |
| 1339 | gi\|4826932 | NP_005029.1 | peptidyl-prolyl cis-trans isomerase D [Homo sapiens] |
| 1340 | gi\|207029439 | NP_001128728.1 | histone-binding protein RBBP4 isoform c [Homo sapiens] |
| 1341 | gi\|171543895 | NP_002964.3 | ataxin-2 [Homo sapiens] |
| 1342 | gi\|51477702 | NP_001003801.1 | SWI/SNF-related matrix-associated actin-dependent regulator of chromatin subfamily D member 3 isoform 2 [Homo sapiens] |
| 1343 | gi\|54607089 | NP_001005914.1 | semaphorin-3B isoform 2 precursor [Homo sapiens] |
| 1344 | gi\|41281447 | NP_055529.2 | intraflagellar transport protein 140 homolog [Homo sapiens] |
| 1345 | gi\|5803102 | NP_006782.1 | mortality factor 4-like protein 1 isoform 1 [Homo sapiens] |
| 1346 | gi\|58218981 | NP_001009955.1 | single-stranded DNA-binding protein 3 isoform c [Homo sapiens] |
| 1347 | gi\|41350201 | NP_037465.2 | epsin-1 isoform c [Homo sapiens] |
| 1348 | gi\|8923417 | NP_060295.1 | poly(ADP-ribose) glycohydrolase ARH3 [Homo sapiens] |
| 1349 | gi\|268370293 | NP_065175.4 | valyl-tRNA synthetase, mitochondrial isoform 2 precursor [Homo sapiens] |
| 1350 | gi\|10257437 | NP_065710.1 | protein tweety homolog 1 isoform 1 [Homo sapiens] |
| 1351 | gi\|165932370 | NP_078858.4 | protocadherin Fat 4 precursor [Homo sapiens] |
| 1352 | gi\|50263044 | NP_116197.4 | leucine-rich repeat neuronal 6A [Homo sapiens] |
| 1353 | gi\|45446740 | NP_001597.2 | ATP-binding cassette sub-family A member 2 isoform a [Homo sapiens] |
| 1354 | gi\|71565154 | NP_000662.3 | alcohol dehydrogenase class-3 [Homo sapiens] |
| 1355 | gi\|156523968 | NP_001609.2 | poly [ADP-ribose] polymerase 1 [Homo sapiens] |
| 1356 | gi\|22748667 | NP_689509.1 | sodium/potassium-transporting ATPase subunit alpha-3 [Homo sapiens] |
| 1357 | gi\|82546843 | NP_004318.3 | breakpoint cluster region protein isoform 1 [Homo sapiens] |
| 1358 | gi\|20149594 | NP_031381.2 | heat shock protein HSP 90-beta [Homo sapiens] |
| 1359 | gi\|5031877 | NP_005564.1 | lamin-B1 isoform 1 [Homo sapiens] |
| 1360 | gi\|31543106 | NP_005572.2 | basal cell adhesion molecule isoform 1 precursor [Homo sapiens] |
| 1361 | gi\|20070125 | NP_000909.2 | protein disulfide-isomerase precursor [Homo sapiens] |
| 1362 | gi\|20986499 | NP_620601.1 | mitogen-activated protein kinase 7 isoform 2 [Homo sapiens] |
| 1363 | gi\|4557871 | NP_001054.1 | serotransferrin precursor [Homo sapiens] |
| 1364 | gi\|124028529 | NP_004810.2 | symplekin [Homo sapiens] |
| 1365 | gi\|15011974 | NP_004714.2 | rho guanine nucleotide exchange factor 2 isoform 3 [Homo sapiens] |
| 1366 | gi\|115298668 | NP_004805.2 | U5 small nuclear ribonucleoprotein 40 kDa protein [Homo sapiens] |
| 1367 | gi\|148922920 | NP_055671.2 | DENN domain-containing protein 4B [Homo sapiens] |
| 1368 | gi\|9257197 | NP_059344.1 | brain-specific angiogenesis inhibitor 1-associated protein 2 isoform 1 [Homo sapiens] |
| 1369 | gi\|38455427 | NP_006421.2 | T-complex protein 1 subunit delta [Homo sapiens] |
| 1370 | gi\|300192933 | NP_006787.2 | AFG3-like protein 2 [Homo sapiens] |
| 1371 | gi\|166795250 | NP_006836.2 | kinesin-like protein KIF2C [Homo sapiens] |
| 1372 | gi\|21396480 | NP_031393.2 | RNA-binding protein Raly short isoform [Homo sapiens] |
| 1373 | gi\|150010639 | NP_056091.1 | ubiquitin carboxyl-terminal hydrolase 22 [Homo sapiens] |
| 1374 | gi\|82659109 | NP_065816.2 | E3 ubiquitin-protein ligase UBR4 [Homo sapiens] |
| 1375 | gi\|151101235 | NP_036593.2 | TERF1-interacting nuclear factor 2 isoform 2 [Homo sapiens] |
| 1376 | gi\|6912524 | NP_036468.1 | nuclear prelamin A recognition factor isoform a [Homo sapiens] |
| 1377 | gi\|7706687 | NP_057421.1 | ena/VASP-like protein [Homo sapiens] |
| 1378 | gi\|37622894 | NP_919442.1 | RING finger protein 126 [Homo sapiens] |
| 1379 | gi\|20521788 | BAA86501.2 | KIAA1187 protein [Homo sapiens] |
| 1380 | gi\|34452732 | NP_065871.2 | phosphatidylinositol 3,4,5-trisphosphate-dependent Rac exchanger 1 protein [Homo sapiens] |
| 1381 | gi\|14249678 | NP_116294.1 | splicing factor 45 [Homo sapiens] |
| 1382 | gi\|209862909 | NP_001129525.1 | transmembrane protein adipocyte-associated 1 isoform 1 [Homo sapiens] |
| 1383 | gi\|6005988 | NP_009030.1 | low molecular weight phosphotyrosine protein phosphatase isoform b [Homo sapiens] |
| 1384 | gi\|9951915 | NP_000678.1 | adenosylhomocysteinase isoform 1 [Homo sapiens] |
| 1385 | gi\|18201905 | NP_000166.2 | glucose-6-phosphate isomerase isoform 2 [Homo sapiens] |
| 1386 | gi\|4504193 | NP_001505.1 | transcription initiation factor IIB [Homo sapiens] |
| 1387 | gi\|8051608 | NP_002125.3 | heme oxygenase 2 [Homo sapiens] |
| 1388 | gi\|193785653 | BAG51088.1 | unnamed protein product [Homo sapiens] |
| 1389 | gi\|19923142 | NP_002256.2 | importin subunit beta-1 [Homo sapiens] |
| 1390 | gi\|156631005 | NP_002803.2 | 26S proteasome non-ATPase regulatory subunit 8 [Homo sapiens] |
| 1391 | gi\|154759259 | NP_003118.2 | spectrin alpha chain, brain isoform 2 [Homo sapiens] |
| 1392 | gi\|4507385 | NP_003186.1 | transcription elongation factor A protein 2 isoform a [Homo sapiens] |
| 1393 | gi\|4507669 | NP_003286.1 | translationally-controlled tumor protein [Homo sapiens] |
| 1394 | gi\|21396500 | NP_003600.2 | HIRA-interacting protein 3 [Homo sapiens] |
| 1395 | gi\|4503523 | NP_003744.1 | eukaryotic translation initiation factor 3 subunit D [Homo sapiens] |
| 1396 | gi\|4504079 | NP_003792.1 | glycosylphosphatidylinositol anchor attachment 1 protein [Homo sapiens] |
| 1397 | gi\|19923191 | NP_003897.2 | 80 kDa MCM3-associated protein [Homo sapiens] |
| 1398 | gi\|52486265 | NP_006020.4 | paraneoplastic antigen Ma1 [Homo sapiens] |
| 1399 | gi\|34365371 | CAE46007.1 | hypothetical protein [Homo sapiens] |
| 1400 | gi\|6912448 | NP_036418.1 | mortality factor 4-like protein 2 [Homo sapiens] |
| 1401 | gi\|148727368 | NP_056510.2 | bromodomain and PHD finger-containing protein 3 [Homo sapiens] |
| 1402 | gi\|14249268 | NP_116076.1 | cysteine and histidine-rich protein 1 isoform 2 [Homo sapiens] |
| 1403 | gi\|7705877 | NP_057269.1 | hematological and neurological expressed 1 protein isoform 1 [Homo sapiens] |
| 1404 | gi\|9910382 | NP_064628.1 | mitochondrial import receptor subunit TOM22 homolog [Homo sapiens] |
| 1405 | gi\|32455273 | NP_006639.3 | serine/threonine-protein kinase WNK2 [Homo sapiens] |
| 1406 | gi\|119964728 | NP_689996.4 | D-2-hydroxyglutarate dehydrogenase, mitochondrial precursor [Homo sapiens] |
| 1407 | gi\|38327632 | NP_001945.3 | epithelial discoidin domain-containing receptor 1 isoform 1 precursor [Homo sapiens] |
| 1408 | gi\|4503065 | NP_001879.1 | mu-crystallin homolog isoform 1 [Homo sapiens] |
| 1409 | gi\|4503725 | NP_000792.1 | peptidyl-prolyl cis-trans isomerase FKBP1A isoform a [Homo sapiens] |
| 1410 | gi\|4505489 | NP_002530.1 | ornithine decarboxylase [Homo sapiens] |
| 1411 | gi\|38679892 | NP_006214.2 | peptidyl-prolyl cis-trans isomerase NIMA-interacting 4 isoform 1 [Homo sapiens] |
| 1412 | gi\|194018537 | NP_002757.2 | phosphoribosyl pyrophosphate synthase-associated protein 1 [Homo sapiens] |
| 1413 | gi\|4506671 | NP_000995.1 | 60S acidic ribosomal protein P2 [Homo sapiens] |
| 1414 | gi\|4506711 | NP_001021.1 | 40S ribosomal protein S27 [Homo sapiens] |
| 1415 | gi\|4507801 | NP_003343.1 | small ubiquitin-related modifier 1 isoform a precursor [Homo sapiens] |
| 1416 | gi\|27501450 | NP_003493.1 | axin-1 isoform a [Homo sapiens] |
| 1417 | gi\|153792768 | NP_067018.2 | claudin-6 [Homo sapiens] |
| 1418 | gi\|151108509 | NP_055674.4 | rho GTPase-activating protein 44 [Homo sapiens] |
| 1419 | gi\|22907052 | NP_006400.2 | actin-related protein 2/3 complex subunit 1A isoform 1 [Homo sapiens] |
| 1420 | gi\|5803167 | NP_006793.1 | splicing factor 3A subunit 3 [Homo sapiens] |
| 1421 | gi\|8922116 | NP_060056.1 | calcium channel flower homolog isoform a [Homo sapiens] |
| 1422 | gi\|41281557 | NP_055736.2 | latrophilin-1 isoform 2 precursor [Homo sapiens] |
| 1423 | gi\|6912634 | NP_036555.1 | 60S ribosomal protein L13a [Homo sapiens] |
| 1424 | gi\|6912440 | NP_036411.1 | zinc finger protein 346 [Homo sapiens] |
| 1425 | gi\|7656938 | NP_055150.1 | EP300-interacting inhibitor of differentiation 1 [Homo sapiens] |
| 1426 | gi\|166197690 | NP_001107562.1 | ephexin-1 isoform 2 [Homo sapiens] |
| 1427 | gi\|75677343 | NP_056359.2 | transmembrane protein 98 [Homo sapiens] |
| 1428 | gi\|27414497 | NP_054872.2 | zinc finger CCCH domain-containing protein 7A [Homo sapiens] |
| 1429 | gi\|8922867 | NP_060792.1 | hypothetical protein LOC55776 [Homo sapiens] |
| 1430 | gi\|40255020 | NP_073593.2 | chromatin modification-related protein MEAF6 [Homo sapiens] |
| 1431 | gi\|16554616 | NP_115865.1 | 28S ribosomal protein S6, mitochondrial [Homo sapiens] |
| 1432 | gi\|41872403 | NP_958800.1 | FAD synthase isoform 2 [Homo sapiens] |
| 1433 | gi\|14150149 | NP_115726.1 | hypothetical protein LOC84310 [Homo sapiens] |
| 1434 | gi\|56786147 | NP_001792.2 | cysteine dioxygenase type 1 [Homo sapiens] |
| 1435 | gi\|55750041 | NP_001931.2 | atrophin-1 [Homo sapiens] |
| 1436 | gi\|194097323 | NP_004083.3 | enoyl-CoA hydratase, mitochondrial precursor [Homo sapiens] |
| 1437 | gi\|116063573 | NP_001447.2 | filamin-A isoform 1 [Homo sapiens] |
| 1438 | gi\|38044288 | NP_937895.1 | gelsolin isoform b [Homo sapiens] |
| 1439 | gi\|4504511 | NP_001530.1 | dnaJ homolog subfamily A member 1 [Homo sapiens] |
| 1440 | gi\|4504897 | NP_002257.1 | importin subunit alpha-2 [Homo sapiens] |
| 1441 | gi\|4505021 | NP_002328.1 | alpha-2-macroglobulin receptor-associated protein precursor [Homo sapiens] |
| 1442 | gi\|93141029 | NP_001035224.1 | paralemmin isoform 2 [Homo sapiens] |
| 1443 | gi\|103485496 | NP_002759.2 | charged multivesicular body protein 1a isoform 2 [Homo sapiens] |
| 1444 | gi\|313760624 | NP_000433.4 | platelet endothelial cell adhesion molecule precursor [Homo sapiens] |
| 1445 | gi\|47132589 | NP_002732.3 | serine/threonine-protein kinase N1 isoform 2 [Homo sapiens] |
| 1446 | gi\|25777602 | NP_002799.3 | 26S proteasome non-ATPase regulatory subunit 2 [Homo sapiens] |
| 1447 | gi\|5453990 | NP_006254.1 | proteasome activator complex subunit 1 isoform 1 [Homo sapiens] |
| 1448 | gi\|104487295 | NP_570923.2 | receptor-type tyrosine-protein phosphatase S isoform 3 precursor [Homo sapiens] |
| 1449 | gi\|4506693 | NP_001012.1 | 40S ribosomal protein S17 [Homo sapiens] |
| 1450 | gi\|156416003 | NP_004159.2 | succinate dehydrogenase [ubiquinone] flavoprotein subunit, mitochondrial precursor [Homo sapiens] |
| 1451 | gi\|5902076 | NP_008855.1 | serine/arginine-rich splicing factor 1 isoform 1 [Homo sapiens] |
| 1452 | gi\|112180565 | AAH61586.1 | TMSB4X protein [Homo sapiens] |
| 1453 | gi\|4507987 | NP_003428.1 | zinc finger protein 136 [Homo sapiens] |
| 1454 | gi\|4503513 | NP_003748.1 | eukaryotic translation initiation factor 3 subunit I [Homo sapiens] |
| 1455 | gi\|23397696 | NP_690902.1 | copine-1 isoform a [Homo sapiens] |
| 1456 | gi\|153791497 | NP_055490.3 | rootletin [Homo sapiens] |
| 1457 | gi\|299829177 | NP_008920.5 | ras GTPase-activating protein 4 isoform 1 [Homo sapiens] |
| 1458 | gi\|5031981 | NP_005796.1 | 26S proteasome non-ATPase regulatory subunit 14 [Homo sapiens] |
| 1459 | gi\|5453547 | NP_006325.1 | noelin isoform 2 [Homo sapiens] |
| 1460 | gi\|95113651 | NP_006532.2 | glutaredoxin-3 [Homo sapiens] |
| 1461 | gi\|31542319 | NP_009194.2 | coatomer subunit epsilon isoform a [Homo sapiens] |
| 1462 | gi\|109891938 | NP_001035951.1 | disks large-associated protein 4 isoform c [Homo sapiens] |
| 1463 | gi\|112363070 | NP_036399.3 | hsp70-binding protein 1 [Homo sapiens] |
| 1464 | gi\|8392875 | NP_037374.1 | transcription factor IIB [Homo sapiens] |
| 1465 | gi\|9790193 | NP_062819.1 | protein reprimo [Homo sapiens] |
| 1466 | gi\|31652259 | NP_116242.2 | PWWP domain-containing protein MUM1 [Homo sapiens] |
| 1467 | gi\|50086624 | NP_291025.3 | integrator complex subunit 4 [Homo sapiens] |

## Patentansprüche

1. Anordnung von Markersequenzen für gynäkologisches Malignom auf einem Träger zur Früherkennung, Diagnose, Prognose, Therapiesteuerung bei gynäkologischem Malignom mit einer oder mehreren verschiedenen Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID. No. 1 - 978.

2. Anordnung von Markersequenzen für gynäkologisches Malignom auf einem Träger zur Analyse von Autoantikörperprofilen assoziiert mit gynäkologischen Malignom mit einer oder mehreren Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID. No. 1 - 978.

3. Anordnung nach einem der vorgehenden Ansprüche wobei 2 oder 3, vorzugsweise 4 oder 5, besonders bevorzugt 7 oder 8 oder mehr unterschiedliche Markersequenzen für gynäkologisches Malignom umfasst sind.

4. Anordnung nach einem der vorgehenden Ansprüche wobei mindestens 2 bis 5 oder 10, vorzugsweise 30 bis 50 Markersequenzen für gynäkologisches Malignom oder 50 bis 100 oder mehr Markersequenzen für gynäkologisches Malignom an oder zu einem zu untersuchenden Patienten bestimmt werden.

5. Anordnung nach einem der vorgehenden Ansprüche, **dadurch gekennzeichnet, dass** die Markersequenzen für gynäkologisches Malignom auf einem festen Träger aufgebracht werden, insbesondere einem Filter, einer Membran, einem magnetischen oder Fluorophor-markierten Kügelchen, einem Silizium-Wafer, Glas, Metall, Kunststoff, einem Chip, einem massenspektrometrischen Target oder einer Matrix.

6. Anordnung nach einem der vorgehenden Ansprüche, **dadurch gekennzeichnet, dass** die Markersequenzen für gynäkologisches Malignom als Klone vorliegen.

7. Assay oder Proteinbiochip umfassend eine Anordnung nach einem der Ansprüche 1 bis 6.

8. Verwendung einer Anordnung nach einem der Ansprüche 1 bis 6 oder eines Assays oder Proteinbiochips nach Anspruch 7 zur Analyse von Autoantikörperprofilen von Patienten, insbesondere zur quantitativen Analyse und/oder zur Überwachung von Veränderungen von Autoantikörperprofilen von Patienten.

9. Diagnostikum (Test Kit) zur Früherkennung und/oder Diagnose von gynäkologischem Malignom und/oder Prognose und/oder Vorhersage des Risikos der Metastasenbildung bei gynäkologischem Malignom umfassend eine Anordnung nach einem der Ansprüche 1 bis 6 oder einen Assay oder Proteinbiochip nach Anspruch 7 und gegebenenfalls weitere Zusatz- und Hilfsstoffe.

10. Diagnostikum (Test Kit) zur Therapieüberwachung und/oder Nachsorge bei gynäkologischem Malignom umfassend eine Anordnung nach einem der Ansprüche 1 bis 6 oder einen Assay oder Proteinbiochip nach Anspruch 7 und gegebenenfalls weitere Zusatz- und Hilfsstoffe.

11. Verfahren zur Früherkennung und Diagnose von gynäkologischem Malignom wobei
a.) eine oder mehrere Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID No. 1 - 978 auf einem Träger aufgebracht werden und
b.) mit Körperflüssigkeit oder Gewebeauszug eines Patienten in Kontakt gebracht werden und
c.) der Nachweis einer Wechselwirkung der Körperflüssigkeit oder Gewebeauszug mit den Markersequenzen für gynäkologisches Malignom aus a.) erfolgt.

12. Verfahren zum Stratifizieren, insbesondere zur Risikostratifizierung, oder zur Therapiesteuerung eines Patienten mit gynäkologischem Malignom, wobei mit Hilfe einer oder mehrerer Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID No. 1 - 978 das Autoantikörperprofil eines Patienten bestimmt und gegebenenfalls überwacht wird.

13. Verfahren nach Anspruch 12, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

14. Target zur Behandlung und Therapie von gynäkologischem Malignom ausgewählt aus der Gruppe der Proteine SEQ ID No. 979 bis 1467, Teilsequenzen dieser Proteine und den für diese Proteine kodierenden Sequenzen, den Sequenzen SEQ ID No. 1 - 489 und SEQ ID. No. 490 - 978 und Teilsequenzen von SEQ ID No 1 - 978 sowie der von SEQ ID No. 1 - 978 kodierten Proteine.

15. Verwendung einer Markersequenz für gynäkologisches Malignom ausgewählt aus der Gruppe der Proteine SEQ ID No. 979 bis 1467, Teilsequenzen dieser Proteine und den für diese Proteine kodierenden Sequenzen, den Sequenzen SEQ ID No. 1 - 489 und SEQ ID. No. 490 - 978 und Teilsequenzen von SEQ ID No 1 - 978 sowie der von SEQ ID No. 1 - 978 kodierten Proteine als Affinitätsmaterial zur Durchführung einer Apherese oder Blutwäsche für Patienten mit gynäkologischem Malignom.

16. Verfahren zur Identifizierung von Markersequenzen für gynäkologisches Malignom umfassend die Schritte
a. Bereitstellung von Markersequenzen auf einem Array,
b. Identifizierung von Markersequenzkandidaten für gynäkologisches Malignom durch vergleichende Analyse der Signale, die bei Kontakt der Markersequenzen mit
Körperflüssigkeit oder Gewebeauszug eines Patienten mit gynäkologischem Malignom und
Körperflüssigkeit oder Gewebeauszug eines Patienten ohne gynäkologischem Malignom gemessen werden,
c. Charakterisierung der Markersequenzkandidaten für gynäkologisches Malignom mit Hilfe eines Proteinbiochips,
d. Auswahl von Markersequenzkandidaten für gynäkologisches Malignom, die ein unterschiedliches Signal bei Patienten mit gynäkologischem Malignom und Patienten ohne gynäkologisches Malignom liefern (Markersequenzen für gynäkologisches Malignom).

17. Verwendung von einer oder mehreren Markersequenzen für gynäkologisches Malignom ausgewählt aus der Gruppe der Proteine SEQ ID No. 979 bis 1467 und/oder Teilsequenzen dieser Proteine und/oder kodiert durch Sequenzen SEQ ID No. 1 - 489 und/oder kodiert durch Sequenzen SEQ ID. No. 490 - 978 und/oder kodiert durch Teilsequenzen von SEQ ID. No. 1 - 978 für Diagnose und Therapie von gynäkologischem Malignom, insbesondere zur Früherkennung von gynäkologischem Malignom, Diagnose von gynäkologischem Malignom, Prognose, beispielsweise des Risikos der Metastasenbildung, Therapiesteuerung, beispielsweise Vorhersage und Überwachung des Ansprechens auf ein Arzneimittel oder eine Therapie, Nachsorge bei gynäkologischem Malignom.

18. Verwendung einer Anordnung nach einem der Ansprüche 1 bis 6 oder eines Assays oder Proteinbiochips nach Anspruch 7 zum Screenen von Substanzen (Wirkstoffen) für gynäkologisches Malignom.

19. Verfahren zur Identifizierung und/oder Charakterisierung einer Substanz (Wirkstoff)für gynäkologisches Malignom wobei
a) eine oder mehrere Markersequenzen für gynäkologisches Malignom ausgewählt aus der Gruppe der Proteine SEQ ID No. 979 bis 1467 und/oder Teilsequenzen dieser Proteine und/oder der Sequenzen SEQ ID No. 1 - 489 und/oder der Sequenzen SEQ ID. No. 490 - 978 und/oder Teilsequenzen von SEQ ID No. 1 - 978 und/oder der durch die Nukeinsäuresequenzen kodierten Proteine
b) mit mindestens einer zu untersuchenden Substanz in Kontakt gebracht werden,
c) ein Bindungserfolg nachgewiesen wird.
